(19) Europäisches Patentamt
European Patent Office
Office européen des brevets

(11) Veröffentlichungsnummer : **0 278 352 B1**

# (12) EUROPÄISCHE PATENTSCHRIFT

(45) Veröffentlichungstag der Patentschrift : **10.04.91 Patentblatt 91/15**

(51) Int. Cl.$^5$ : **C07D 307/94,** C07D 405/06, C07D 413/06, C07D 417/06, A01N 43/12

(21) Anmeldenummer : **88101375.9**

(22) Anmeldetag : **01.02.88**

## (54) Aminomethyltetrahydrofurane.

Die Akte enthält technische Angaben, die nach dem Eingang der Anmeldung eingereicht wurden und die nicht in dieser Patentschrift enthalten sind.

(30) Priorität : **10.02.87 DE 3703972**
**11.09.87 DE 3730499**

(43) Veröffentlichungstag der Anmeldung : **17.08.88 Patentblatt 88/33**

(45) Bekanntmachung des Hinweises auf die Patenterteilung : **10.04.91 Patentblatt 91/15**

(84) Benannte Vertragsstaaten : **BE CH DE FR GB IT LI NL**

(56) Entgegenhaltungen : **EP-A- 0 158 922**

(56) Entgegenhaltungen : **TETRAHEDRON LETTERS, Band 28, Nr. 35, Juli 1987, Seiten 4011-4014, Pergamon Press Ltd, Oxford, GB; G.A. Kraus et al.: "Alkoxy radicals in organic synthesis. A novel approach to spiroketals"**
**Heterocycles, 1985, 23 (8) 2035-9**

(73) Patentinhaber : **BAYER AG**
**W-5090 Leverkusen 1 Bayerwerk (DE)**

(72) Erfinder : **Weissmüller, Joachim, Dr.**
**Carl-Langhans-Strasse 53**
**W-4019 Monheim (DE)**
Erfinder : **Berg, Dieter, Dr.**
**Gellertweg 27**
**W-5600 Wuppertal 1 (DE)**
Erfinder : **Dutzmann, Stefan, Dr.**
**Leinenweberweg 33**
**W-4000 Duesseldorf 13 (DE)**
Erfinder : **Reinecke, Paul, Dr.**
**Steinstrasse 8**
**W-5090 Leverkusen 3 (DE)**

**EP 0 278 352 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Aminomethyltetrahydrofurane, mehrere Verfahren zu ihrer Herstellung, sowie ihre Verwendung als Schädlingsbekämpfungsmittel.

Es ist bereits bekannt, daß bestimmte Aminomethyltetrahydrofurane, wie beispielsweise das 2-(4-Chlorphenyl)-5-(3,5-dimethylpiperidin-1-yl-methyl)-tetrahydrofuran fungizide Eigenschaften besitzen (vgl. DE-OS 34 13 996).

Die Wirksamkeit dieser vorbekannten Verbindungen ist jedoch insbesondere beiniedrigen Aufwandmengen und Konzentrationen nicht in allen Anwendungsgebieten völlig zufriedenstellend.

Bekannt ist weiterhin das 2-Bromomethyl- bzw, 2-Iodomethyl-1-oxa-spiro[4,4]-nonan (vgl. Heterocycles 1985, 23 (8), 2035-9).

Es wurden neue Aminomethyltetrahydrofurane der allgemeinen Formel (I),

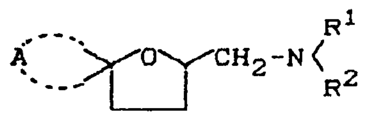 (I)

in welcher

A für eine jeweils gegebenenfalls einfach bis mehrfach, gleich oder verschieden substituierte zweifach verknüpfte Alkylen- oder Alkenylenkette mit jeweils 3 bis 18 Kohlenstoffatomen steht, wobei als Substituenten jeweils infrage kommen :

jeweils geradkettiges oder verzweigtes Alkyl, Alkoxy, Alkylthio, Alkoxycarbonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einselnen geradkettigen oder verzweigten Alkylteilen ;

jeweils gegebenenfalls einfach oder mehrfach, gleich oder verschieden durch Alkyl mit 1 bis 4 Kohlenstoffatomen und/oder Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder versweigten Alkylteil ;

jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes jeweils zweifach verknüpftes Alkylen oder Alkenylen mit jeweils bis zu 5 Kohlenstoffatomen sowie jeweils gegebenenfalls einfach oder mehrfach, gleich oder verschieden substituiertes einfach verknüpftes Aryl oder zweifach verknüpftes Arylen mit jeweils 6 bis 10 Kohlenstoffatomen, wobei als Substituenten jeweils infrage kommen : Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen und

$R^1$ und $R^2$ unabhängig voneinander jeweils für Wasserstoff ; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für jeweils geradkettiges oder verzweigtes Dioxolanylalkyl,Oxolanylalkyl oder Dioxanylalkyl mit jeweils 1 bis 4 Kohlenstoff atomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil stehen, wobei als Substituenten jeweils infrage kommen:

Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen ; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten jeweils infrage kommen :

Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten

kann, wobei als Substituenten infrage kommen ; jeweils geradkettiges oder verzweigtes Alkyl oder Hydroxyalkyl mit jeweils 1 bis 4 Kohlenstoffatomen

sowie deren pflanzenverträgliche Säureadditionssalze gefunden.

Die Verbindungen der Formel (I) können als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen Aminomethyltetrahydrofurane der allgemeinen Formel (I),

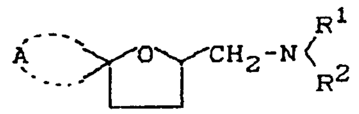
(I)

in welcher für eine jeweils gegebenenfalls einfach bis mehrfach, gleich oder verschieden substituierte zweifach verknüpfte Alkylen- oder Alkenylenkette mit jeweils 3 bis 18 Kohlenstoffatomen steht, wobei als Substituenten jeweils infrage kommen :

jeweils geradkettiges oder verzweigtes Alkyl, Alkoxy, Alkylthio, Alkoxycarbonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einzelnen geradkettigen oder verzweigten Alkylteilen ;

jeweils gegebenenfalls einfach oder mehrfach, gleich oder verschieden durch Alkyl mit 1 bis 4 Kohlenstoffatomen und/oder Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil ;

jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes jeweils zweifach verknüpftes Alkylen oder Alkenylen mit jeweils bis zu 5 Kohlenstoffatomen sowie jeweils gegebenenfalls einfach oder mehrfach, gleich oder verschieden substituiertes einfach verknüpftes Aryl oder zweifach verknüpftes Arylen mit jeweils 6 bis 10 Kohlenstoffatomen, wobei als Substituenten jeweils infrage kommen : Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen und

$R^1$ und $R^2$ unabhängig voneinander jeweils für Wasserstoff ; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Mydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für jeweils geradkettiges oder verzweigtes Dioxolanylalkyl Oxolanylalkyl oder Dioxanylalkyl mit jeweils 1 bis 4 Kohlenstoffatomen im Alkylteil oder für jeweils gegebenenfalls in Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil stehen, wobei als Substituenten jeweils infrage kommen:

Halogen sowie jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen ; außerdem für jeweils gegebenenfalls einfach bis mehrfach, gleich oder verschieden im Arylteil substituiertes Arylalkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw, Alkenylteil stehen, wobei als Arylsubstituenten jeweils infrage kommen :

Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom, insbesondere Stickstoff, Sauerstoff oder Schwefel enthalten kann, wobei als Substituenten infrage kommen :

jeweils geradkettiges oder verzweigtes Alkyl oder Hydroxyalkyl mit jeweils 1 bis 4 Kohlenstoffatomen

sowie deren pflanzenvertägliche Säureadditionssalze erhält, wenn man

(a) substituierte Tetrahydrofurane der Formel (II),

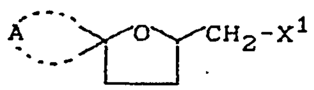

(II)

in welcher

A die oben angegebene Bedeutung hat und

$X^1$ für eine elektronenanziehende Abgangsgruppe steht,

mit Aminen der Formel (III),

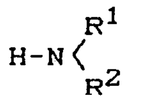

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels umsetzt, oder wenn man

(b) die nach Verfahren (a) erhältlichen Aminomethyltetrahydrofurane der Formel (Ia),

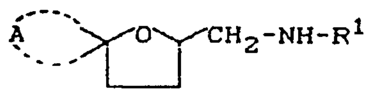

(Ia)

in welcher

$R^1$ und A die oben angegebene Bedeutung haben,

mit Alkylierungsmitteln der Formel (V),

$$R^{2-1} - X^2 \quad (V)$$

in welcher

$R^{2-1}$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen Alkoxyalkyl oder Dialkoxyalkyl mit 1 bis 6 Kohlenstoffatomen oder Hydroxyalkoxyalkyl mit 2 bis 6 Kohlenstoffatomen in den einzelnen Alkylteilen ; für jeweils geradkettiges oder verzweigtes Dioxolanylalkyl, Oxolanylalkyl oder Dioxanylalkyl mit jeweils 1 bis 4 Kohlenstoffatomen im Alkylteil ; für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil, wobei als Substituenten jeweils infrage kommen : Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen ; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl- bzw. Alkenylteil, wobei als Arylsubstituenten jeweils infrage kommmen : Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen und

$X^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls anschließend eine Säure addiert.

Schließlich wurde gefunden, daß die neuen Aminomethyltetrahydrofurane der allgemeinen Formel (I) eine Wirkung gegen Schädlinge, insbesondere gegen pilzliche Schädlinge besitzen.

Überraschenderweise zeigen die erfindungsgemäßen Aminomethyltetrahydrofurane der allgemeinen Formel (I) eine bessere fungizide Wirksamkeit als die aus dem Stand der Technik bekannten Aminomethyltetrahydrofurane, wie beispielsweise das 2-(4-Chlorphenyl)-5-(3,5-dimethylpiperidin-1-yl-methyl)-tetrahydrofuran, welches chemisch und wirkungsmäßig naheliegende Verbindungen sind.

Die erfindungsgemäßen Aminomethyltetrahydrofurane sind durch die Formel (I) allgemein definiert. Bevorzugt sind Verbindungen der Formel (I), bei welchen

A für jeweils gegebenenfalls ein- bis fünffach, gleich oder verschieden substituiertes 1,4-Butylen, 1,5-Pentylen, 1,6-Hexylen, 1,5-Pent-1-enylen, 1,5-Pent-2-enylen oder 1,11-Undecylen steht, wobei als Substituenten jeweils infrage kommen :

Methyl, Ethyl, n- oder i-Propyl, n-, i-, s- oder t-Butyl, n- oder i-Pentyl, n- oder i-Hexyl, n- oder i-Heptyl, n- oder i-Octyl, n- oder i-Nonyl, Methoxy, Ethoxy, n- oder i-Propoxy, n-, i-, s- oder t-Butoxy, Methylthio, Methoxycarbonyl, Ethoxycarbonyl, Trifluormethyl, Trifluormethoxy, Trifluormethylthio, Trimethylsilyl, jeweils gegebenenfalls ein- bis dreifach, gleich oder verschieden durch Fluor, Chlor, Brom, Methyl, Ethyl, n- oder i-Propyl und/oder t-Butyl substituiertes Cyclohexyl, Cyclohexylmethyl, Cyclohexylethyl oder Cyclohexylpropyl, jeweils gegebenenfalls ein- bis dreifach, gleich oder verschieden durch Methyl, Ethyl, n- oder iPropyl, n-, i-, s- oder t-Butyl, Trifluormethyl und/oder Trifluormethoxy substituiertes Methylen, 1,2-Ethylen, 1,3-Propylen, 1,4-Butylen oder 1,4-Butenylen, oder jeweils gegebenenfalls ein- bis dreifach, gleich oder verschieden substituiertes Phenyl oder o-Phenylen, wobei als Substituenten jeweils infrage kommen : Fluor, Chlor, Brom, Cyano, Nitro, Methyl, Ethyl, n- oder i-Propyl, n-, i-, s- oder t-Butyl, Methoxy, Ethoxy, Trifluormethyl, Trifluormethoxy, Trifluormethylthio oder Methoximinomethyl und

$R^1$ und $R^2$ unabhängig voneinander jeweils für Wasserstoff, Methyl, Ethyl, n- oder i-Propyl, n-, i-, s- oder t-Butyl, n- oder i-Pentyl, n-oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Dioxolanylmethyl, Dioxolanylethyl, Oxolanylmethyl, Oxolanylethyl, Dioxanylmethyl, Dioxanylethyl, für jeweils gegebenenfalls ein- bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen : Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

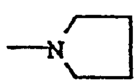 ; 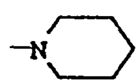 ; 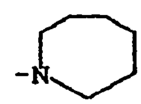 ; 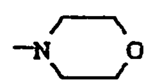

oder 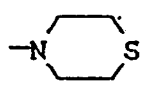

stehen, wobei als Substituenten infrage kommen : Methyl, Ethyl oder Hydroxymethyl.

Ganz besonders bevorzugt sind Verbindungen der Formel (I), bei welchen

A gemeinsam mit dem Kohlenstoffatom, an welches es gebunden ist, für ein gegebenenfalls ein- bis dreifach, gleich oder verschieden substituiertes carbocyclisches Ringsystem der Formel

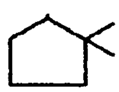 ; 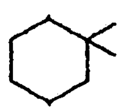 ; 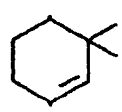 ; 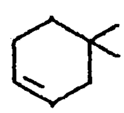 ; 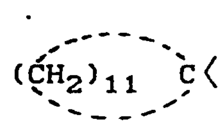

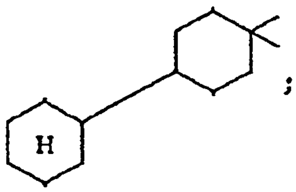 ; 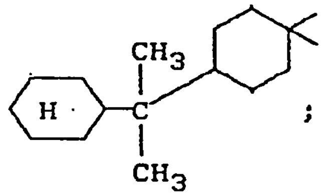 ; 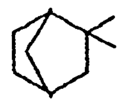 ;

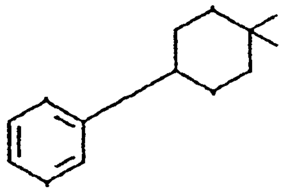 ; 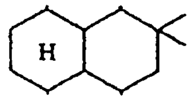 ; 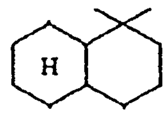

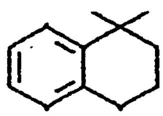 ; 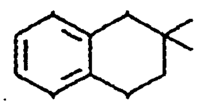 ; 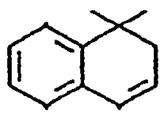 oder

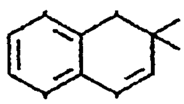 steht,

wobei als Substituenten in den cycloaliphatischen Ringen jeweils infrage kommen : Methyl, Ethyl, noder i-Propyl, n-, i-, s- oder t-Butyl, n- oder i- Pentyl, Trifluormethyl, Trifluormethoxy, Methoxycarbonyl, Ethoxycarbonyl oder Trimethylsilyl und wobei als Substituenten in den aromatischen Ringen jeweils infrage kommen : Fluor, Chlor, Brom, Methyl, Ethyl, n- oder i-Propyl, n-, i-, s- oder t-Butyl, Methoxy, Ethoxy, Trifluormethyl, Trifluormethoxy oder Trifluormethylthio und

$R^1$ und $R^2$ unabhängig voneinander jeweils für Wasserstoff, Methyl, Ethyl, n- oder i-Propyl, n-, i-, s- oder t-Butyl, n- oder i-Pentyl, n-oder i-Hexyl, Allyl, n- oder i-Butenyl, n-oder iPentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxy-, ethyl, Dioxolanylmethyl, Dioxolanylethyl, Oxolanylmethyl, Oxolanylethyl, Dioxanylmethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl stehen oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

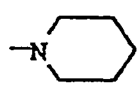 ; 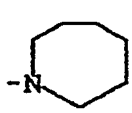 ; 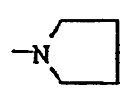 oder 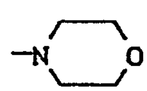

stehen,

wobei als Substituenten infrage kommen : Methyl, Ethyl, Hydroxymethyl,

Bevorzugte erfindungsgemäße Verbindungen sind auch Additionsprodukte aus Säuren und denjenigen Aminomethyltetrahydrofuranen der Formel (I), in denen die Substituenten A, $R^1$ und $R^2$ die Bedeutungen haben, die bereits für diese Substituenten genannt wurden.

Zu den Säuren die addiert werden können, gehören vorzugsweise Halogenwasserstoffsäuren, wie z.B. Chlorwasserstoffsäure und die Bromwasserstoffsäure, insbesondere die Chlorwasserstoffsäure, ferner Phosphorsäure, Salpetersäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie p-ToluoLsulfonsäure und 1,5-NaphthaLindisulfonsäure sowie Saccharin.

Im einzelnen seien außer den bei den Herstellungsbeispielen genannten Verbindungen die folgenden Aminomethyltetrahydrofurane der allgemeinen formel (I) genannt :

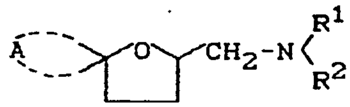

(I)

| 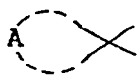 | 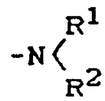 |
|---|---|
| 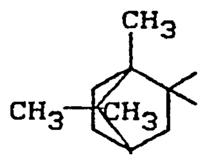 | 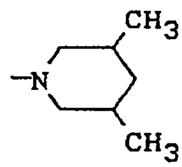 |
| 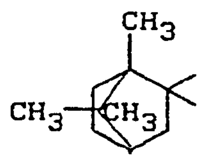 | 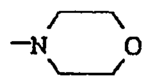 |
| 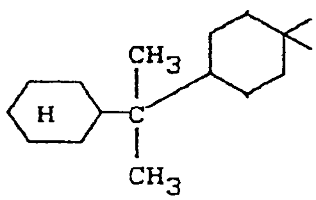 | 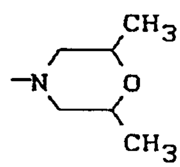 |
| 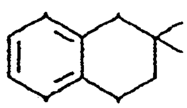 | $-NH-(CH_2)_3-OCH_3$ |

| 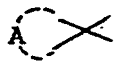 | 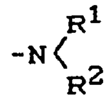 |
|---|---|
| 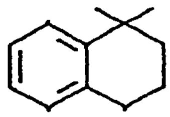 | $-NH-(CH_2)_3-OC_2H_5$ |
| 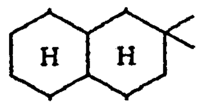 | 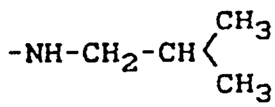 |
| 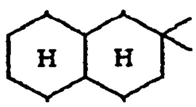 | 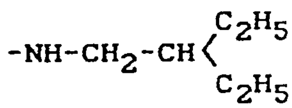 |
| 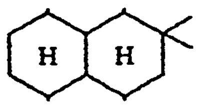 | 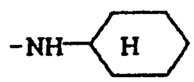 |
| 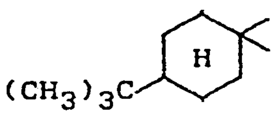 | $-NH-(CH_2)_3-OCH_3$ |
| 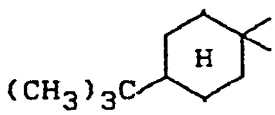 | $-NH-(CH_2)_2-O-(CH_2)_2-OH$ |
| 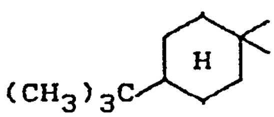 | 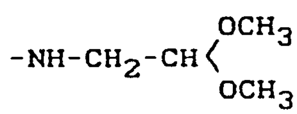 |
| 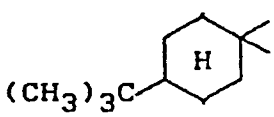 | 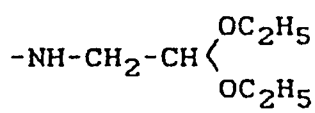 |

| 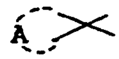 | 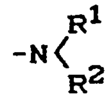 |
| --- | --- |
| 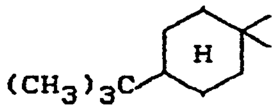 | 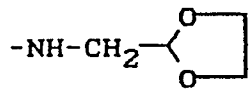 |
| 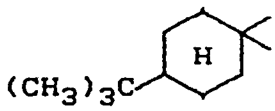 | 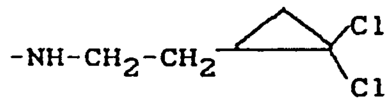 |
| 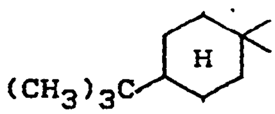 | $-NH-C_3H_7$ |
| 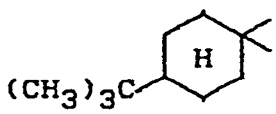 | 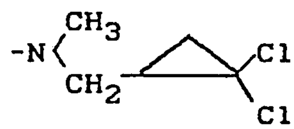 |
| 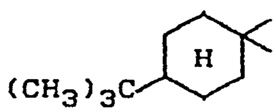 | 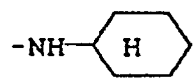 |
| 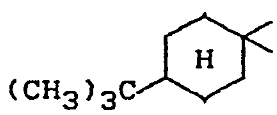 | 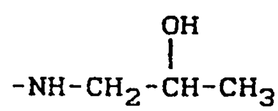 |
| 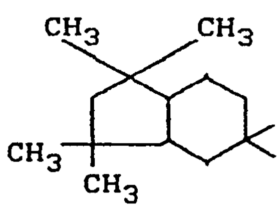 | 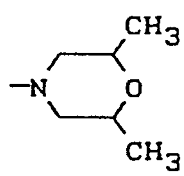 |

| 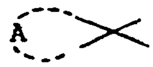 | 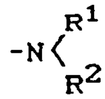 |
|---|---|
| 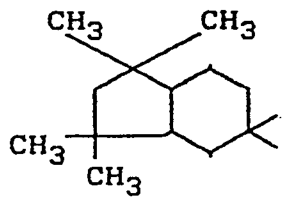 | 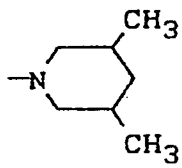 |
| 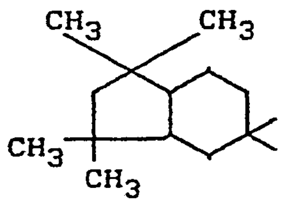 | $-NH-(CH_2)_3-OCH_3$ |
| 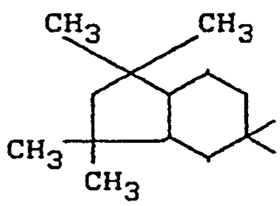 | $-NH-(CH_2)_3-OC_2H_5$ |
| 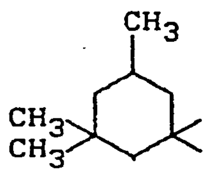 | 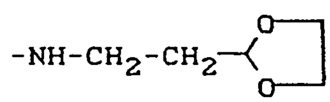 |
| 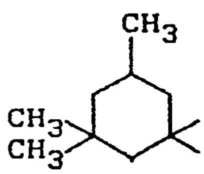 | 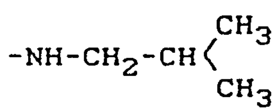 |

| 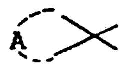 | 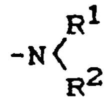 |
|---|---|
| 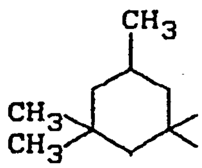 | 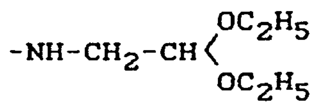 |
| 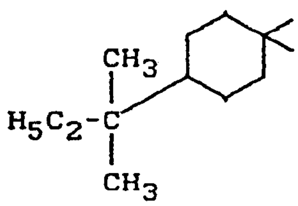 | $-NH-(CH_2)_3-OCH_3$ |
| 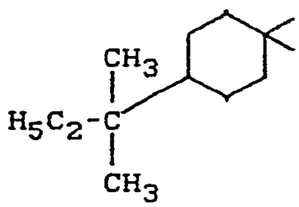 | $-NH(CH_2)_2-OCH_3$ |
| 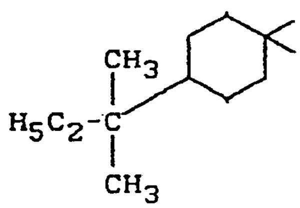 | $-NH-(CH_2)_2-O-(CH_2)_2-OH$ |
| 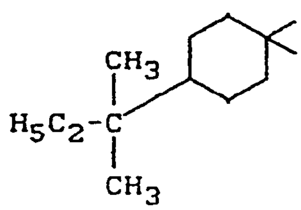 | 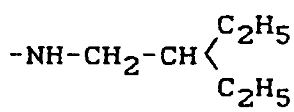 |

| 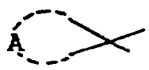 | 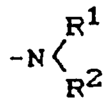 |
|---|---|
| 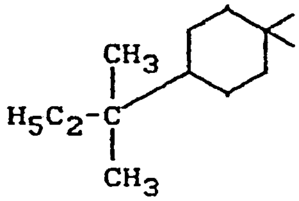 | 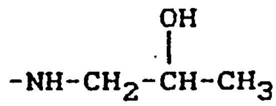 |
| 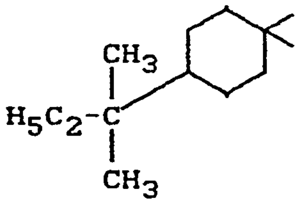 | 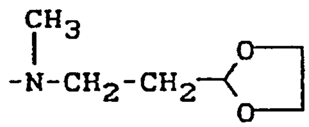 |
| 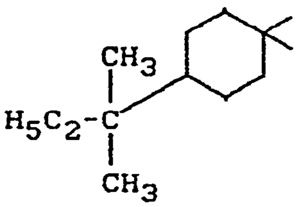 | $-NH-C_4H_9-n$ |
| 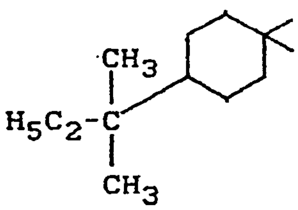 | 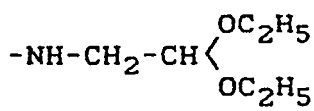 |
| 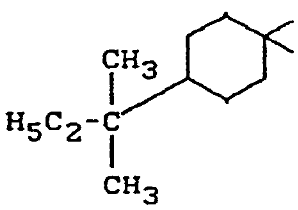 | 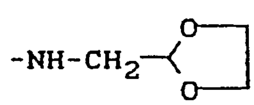 |

| 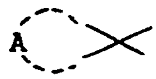 | 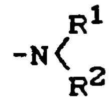 |
|---|---|
| 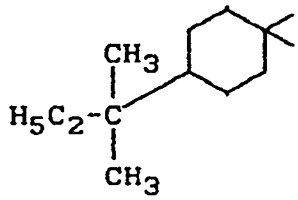 | 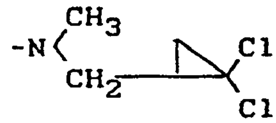 |
| 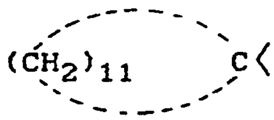 | 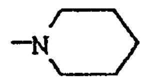 |
| 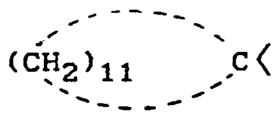 | 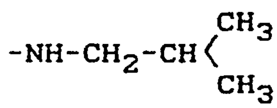 |
| 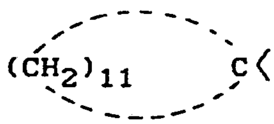 | 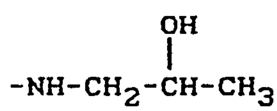 |
| 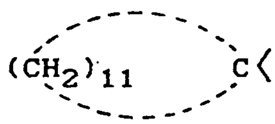 | $-NH-(CH_2)_2-OCH_3$ |
| 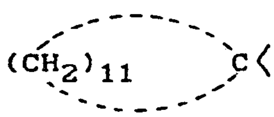 | $-NH-(CH_2)_2-O-(CH_2)_2-OH$ |
| 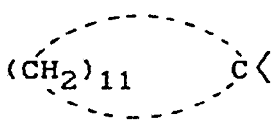 | 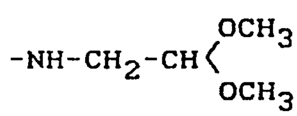 |
| 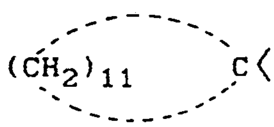 | $-NH-C_3H_7-n$ |

| 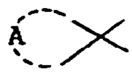 | 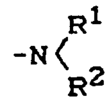 |
| --- | --- |
| 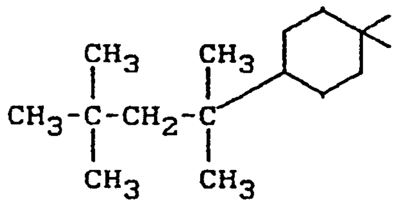 | 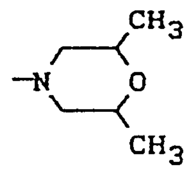 |
| 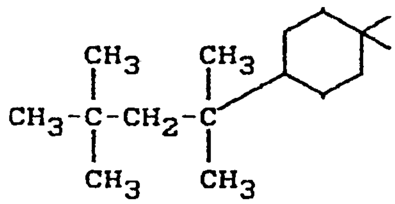 | 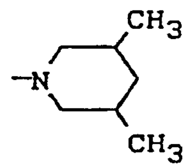 |
| 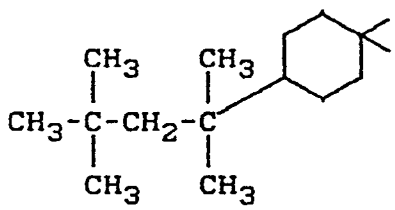 | 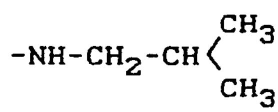 |
| 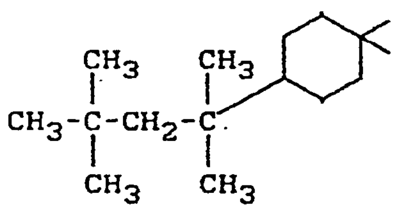 | $-NH-(CH_2)_3-OC_2H_5$ |
| 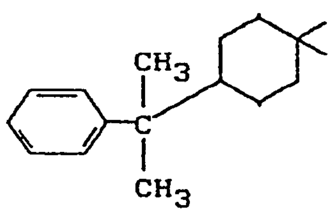 | 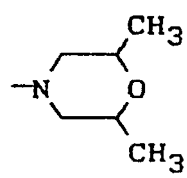 |

| 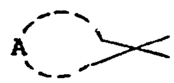 | 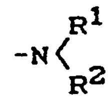 |
|---|---|
| 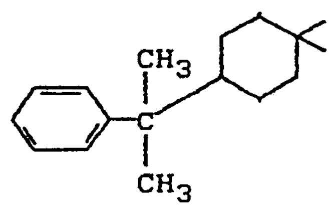 | 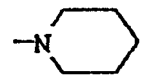 |
| 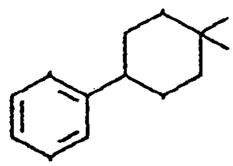 | 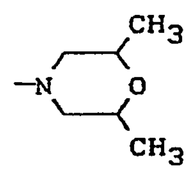 |
| 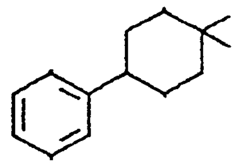 | 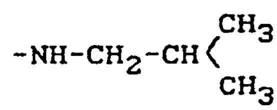 |

Verwendet man beispielsweise 2-Brommethyl-1-oxaspiro[4,5]decan und Piperidin als Ausgangsstoffe, so läßt sich der Reaktionsablauf des erfindungsgemäßen Verfahrens (a) durch das folgende Formelschema darstellen :

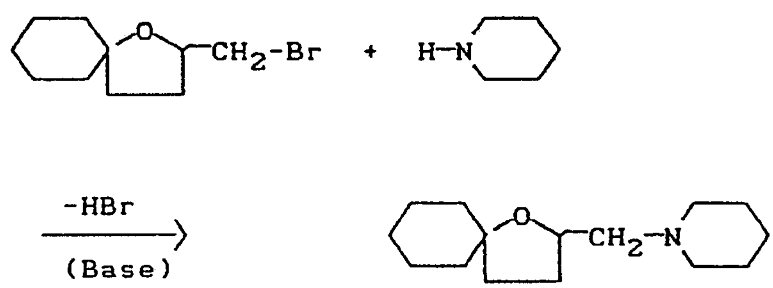


Verwendet man beispielsweise 8-t-Butyl-2-(N-cyclohexylaminomethyl)-1-oxaspiro[4,5]decan und Allylbromid als Ausgangsstoffe, so läßt sich der Reaktionsablauf des erfindungsgemäßen Verfahrens (b) durch das folgende Formelschema darstellen :

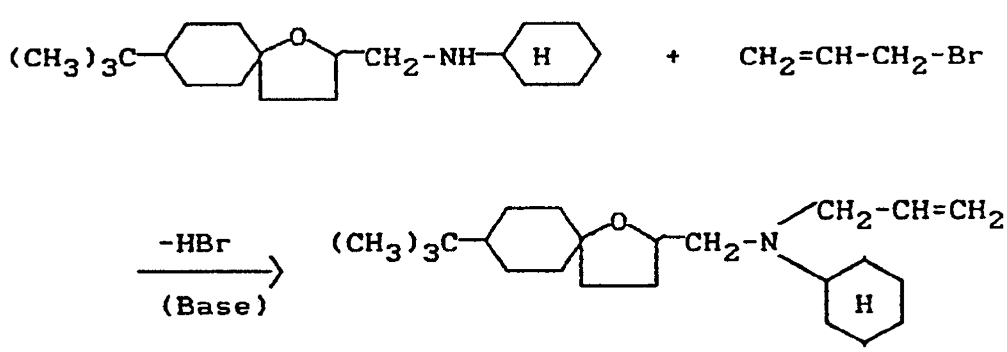


Die zur Durchführung des erfindungsgemäßen Verfahrens (a) als Ausgangsstoffe benötigten substituierten

Tetrahydrofurane sind durch die Formel (II) allgemein definiert. In dieser Formel (II) steht A vorzugsweise für diejenigen Reste, die bereits im Zusammenhang mit der Beschreibung der erfindungsgemäßen Stoffe der Formel (I) als bevorzugt für diese Substituenten genannt wurden.

$X^1$ steht vorzugsweise für Halogen, insbesondere für Chlor oder Brom oder für gegebenenfalls substituiertes Alkylsulfonyloxy mit 1 bis 4 Kohlenstoffatnmen oder gegebenenfalls ein- bis mehrfach durch Alkyl mit 1 bis 4 Kohlenstoffatnmen substituiertes Arylsulfonyloxy, wie beispielsweise Methansulfnnyloxy oder p-Tolunlsulfonyloxy,

Die substituierten Tetrahydrofurane der Formel (II) sind teilweise bekannt (vgl. z.B. Heterocycles 23, 2035-2039 [1985]).

Noch nicht bekannt sind substituierte Tetrahydrofurane der Formel (IIa),

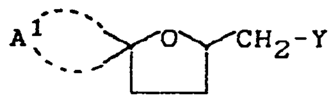

(IIa)

in welcher

$A^1$ für eine jeweils gegebenenfalls einfach bis mehrfach, gleich oder verschieden substituierte zweifach vereknüpfte Alkylen- oder Alkenylenkette mit jeweils 3 bis 18 Kohlenstoffatomen steht, wobei als Substituenten genannt seien :

jeweils geradkettiges oder verzweigtes Alkyl, Alkoxy, Alkylthio, Alkoxycaebonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einzelnen geradkettigen oder verzweigten Alkylteilen ;

jeweils gegebenenfalls einfach bis mehrfach, gleich oder verschieden durch Alkyl mit 1 bis 4 Kohlenstoffatomen und Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil ;

jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes, jeweils zweifach verknüpftes Alkylen oder Alkenylen mit jeweils bis zu 5 Kohlenstoffatomen, jeweils gegebenenfalls einfach bis mehrfach, gleich oder verschieden substituiertes, einfach verknüpftes Aryl oder zweifach verknüpftes Arylen mit jeweils 6 bis 10 Kohlenstoffatomen, wobei als Substituenten genannt seien : Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen,

Y für Halogen oder für jeweils gegebenenfalls substituiertes Alkylsulfonyloxy oder Arylsulfonyloxy steht,

mit Ausnahme der Verbindungen, bei welchen $A^1$ für eine 1,4-Butandiylkette steht und gleichzeitig Y für Brom oder lod steht.

Der Alkylsulfonyloxyrest kann 1 bis 4 Kohlenstoffatome enthalten und gegebenenfalls ein- bis mehrfach substituiert sein durch Halogen und der Arylsulfonyloxyrest steht vorzugsweise für einen gegebenenfalls durch Alkyl mit 1 bis 4 Kohlenstoffatomen substituierten Phenylsulfonyloxyrest.

Man erhält die noch nicht bekannten substituierten Tetrahydrofurane der Formel (IIa) in Analogie zu bekannten Verfahren (vgl, z.B. DE-OS 34 13 996) beispielsweise, indem man die allgemein bekannten cyclischen Ketone der Formel (VI),

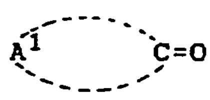

(VI)

in welcher

$A^1$ die oben angegebene Bedeutung hat,

zunächst in einer ersten Stufe mit Buten-1-yl-4magnesiumbromid der Formel (VII)

$$CH_2 = CH - CH_2 - CH_2 - Mg - Br \quad (VII)$$

in Gegenwart eines Verdünnungsmittels, wie beispielsweise Diethylether oder Tetrahydrofuran, bei Temperaturen zwischen –20°C und +50°C umsetzt (vgl. hierzu auch Tetrahedron Lett. 26, 127-130 [1985] ; Bull.

Soc. Chim. Fr. 12, Pt. 2, 3377-3381 [1973] oder die Herstellungsbeispiele) und die so erhältlichen Carbinole der Formel (VIII),

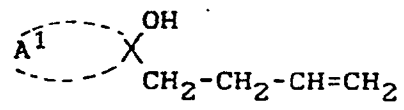

(VIII)

in welcher

A¹ die oben angegebene Bedeutung hat,

mit einem Halogenierungsmittel, wie beispielsweise elementarem Brom, in Gegenwart von Chinolin oder mit NBromsuccinimid, gegebenenfalls in Gegenwart eines Verdünnungsmittels, wie beispielsweise Chloroform, bei Temperaturen zwischen –20°C und +80°C umsetzt (vgl. hierzu auch DE-OS 34 13 996), oder wenn man Trihydroxyverbindungen der Formel IX)

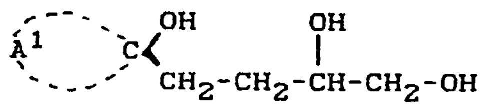

(IX)

in welcher

A¹ die oben angegebene Bedeutung hat, mit Säuren, wie beispielsweise Schwefelsäure oder Phosphorsäure, in üblicher Weise cyclisiert (vgl. z.B. Houben-Weyl "Methoden der organischen Chemie" Band VI/3 S. 528 ; 4. Auflage, Thieme Verlag Stuttgart oder Khim. Geterotsikl. Soedin. Sb, No. 2, 15-17, [1970] bzw. C.A. 77, 48144 g) und die so erhältlichen Hydroxymethyltetrahydrofurane der Formel (X),

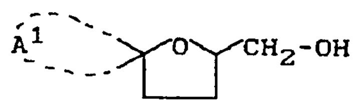

(X)

in welcher

A¹ die oben angegebene Bedeutung hat,

in einer 2. Stufe mit Sulfonsäurehalogeniden der Formel (XI),

$$R^3 - SO_2 - Hal \quad (XI)$$

in welcher

R³ für gegebenenfalls durch Halogen substituiertes Alkyl oder gegebenenfalls durch Alkyl mit 1 bis 4 Kohlenstoffatomen substituiertes Aryl, insbesondere für Methyl, Trifluormethyl oder p-Tolyl steht und

Hal für Halogen, insbesondere für Chlor, Brom oder Iod steht,

gegebenenfalls in Gegenwart eines Verdünnungsmittels, wie beispielsweise Dichlormethan, und gegebenenfalls in Gegenwart eines Säurebindemittels, wie beispielsweise Pyridin, bei Temperaturen zwischen 0° und 120°C umsetzt.

Die Carbinole der Formel (VIII) erhält man altenativ auch, wenn man die Ketone der Formel (VI) in prinzipiell bekannter Weise beispielsweise mit Trimethylsulfoniumylid epoxidiert und anschließend mit Allylmagnesiumbromid umsetzt (vgl. z.B. J, Am. Chem. Soc, 87, 1363- 1364 [1965] oder Heterocycles 8, 397 [1977]).

Die Sulfonsäurehalogenide der Formel (XI) sind allgemein bekannte Verbindungen der organischen Chemie.

Die Trihydroxyverbindungen der Formel (IX) sind bekannt oder erhältlich in Analogie zu bekannten Verfahren (vgl. z.B, Bull. Soc. Chim. France 1964, 564-569 ; Belg, Pat, 631.243 vom 04,11.1963 ; Belg. Pat. 631.242 vom 04.11.1963 ; GB 1.036.087 vom 13.07,1966 oder FR 1,334.968 vom 16.08,1963).

Die zur Durchführung des erfindungsgemäßen Verfahrens (a) weiterhin als Ausgangsstoffe benötigten Amine sind durch die Formel (III) allgemein definiert. In dieser Formel (III) stehen R¹ und R² vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) als Ausgangsstoffe benötigten Aminomethyltetrahydrofurane sind durch die Formel (Ia) allgemein definiert. In dieser Formel (Ia) stehen A und R¹

vorzugsweise für diejenigen Reste, die bereits im Zusammenhang mit der Beschreibung der erfindungsgemäßen Stoffe der Formel (I) als bevorzugt für diese Substituenten genannt wurden,

Die Aminomethyltetrahydrofurane der Formel (Ia) sind erfindungsgemäße Verbindungen und erhältlich mit Hilfe des erfindungsgemäßen Verfahrens (a).

Die zur Durchführung des erfindungsgemäßen Verfahrens (b) weiterhin als Ausgangsstoffe benötigten Alkylierungsmittel sind durch die Formel (V) allgemein defi-niert. In dieser Formel (V) steht

$R^{2-1}$ bevorzugt für Methyl, Ethyl, n- oder i-Propyl, n-, i-, s- oder t-Butyl, n- oder i-Pentyl, n- oder i-Hexyl, n- oder i-Heptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Mydroxyethoxyethyl, Dimethoxyethyl, Dimethoxypropyl, Diethoxyethyl, Dioxolanylmethyl, Dioxolanylethyl, Oxolanylmethyl, Oxolanylethyl, Dioxanylmethyl, Dioxanylethyl, für gegebenenfalls jeweils ein- bis fünffach, gleich oder verschieden durch Fluor, Chlor, Brom, Methyl, Ethyl, n- oder i-Propyl, n-, i-, s-und/oder t-Butyl, substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Benzyl oder Phenylethyl, wobei als Substituenten jeweils infrage kommen : Fluor, Chlor, Brom, Cyano, Nitro, Methyl, Ethyl, n- oder iPropyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl.

$R^{2-1}$ steht besonders bevorzugt für Methyl, Ethyl, n- oder i-Propyl, n-, i-, s- oder t-Butyl, n- oder i-Pentyl, n- oder i-Hexyl, Allyl, n- oder i- Butenyl, n- oder i-Pentenyl, Propargyl, n- oder i- Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Dioxolanylmethyl, Dioxolanylethyl, Oxolanylmethyl, Oxolanylethyl, Dioxanylmethyl, Cyclopropylmethyl, Dichlorcyclopropylmethyl, Dimethylcyclopropylmethyl, Dichlordimethylcyclopropylmethyl, Cyclopentyl, Cylohexyl oder Cyclohexylmethyl.

$X^2$ steht vorzugsweise für Halogen, insbesondere für Chlor, Brom oder lod oder für jeweils gegebenenfalls durch Halogen substituiertes Alkylsulfonyloxy oder Alkoxysulfonyloxy mit jeweils 1 bis 4 Kohlenstoffatomen und gegebenenfalls 1 bis 9 Halogenatomen oder gegebenenfalls durch Alkyl mit 1 bis 4 Kohlenstoffatomen substituiertes Arylsulfonyloxy, wie beispielsweise Methansulfonyloxy, Methoxysulfonyloxy oder p-Toluolsulfonyloxy,

Die Alkylierungsmittel der Formel (V) sind ebenfalls allgemein bekannte Verbindungen der organischen Chemie oder erhältlich in Analogie zu allgemein bekannten Verfahren.

Als Verdünnungsmittel zur Durchführung der erfindungsgemäßen Verfahren (a) und (b) kommen inerte organische Lösungsmittel oder wäßrige Systeme infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff ; Ether, wie Diethylether, Dioxan, Tetrahydrofuran oder Ethylenglykoldimethyl oder -diethylether ; Ketone, wie Aceton oder Butanon ; Nitrile, wie Acetonitril oder Propionitril ; Amide, wie Dimethylformamid, Dimethylacetamid, N-Methylformanilid, N-Methylpyrrolidon oder Hexamethylphosphorsäuretriamid ; Ester, wie Essigsäureethylester oder Sulfoxide, wie Dimethylsulfoxid.

Die erfindungsgemäßen Verfahren (a) und (b) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt : Tetrabutylammoniumiodid, Tetrabutylammoniumbromid, Tributyl-methylphosphoniumbromid, Trimethyl-$C_{13}$/$C_{15}$-alkylammoniumchlorid, Dibenzyldimethyl-ammoniummethylsulfat, Dimethyl-$C_{12}$/$C_{14}$- alkylbenzylammoniumchlorid, Tetrabutylammoniuhydroxid, 15-Krone-5, 18-Krone-6, Triethylbenzylammoniumchlorid, Trimethylbenzylammoniumchlorid. Es ist auch möglich die erfindungsgemäßen Verfahren (a) und (b) ohne Zusatz eines Lösungsmittels durchzuführen.

Als Säurebindemittel zur Durchführung der erfindungsgemäßen Verfahren (a) und (b) kommen alle üblicherweise verwendbaren anorganischen und organischen Basen infrage. Vorzugsweise verwendet man Alkalimetallhydroxide, -carbonate oder -hydrogencarbonate, wie beispielsweise Natriumhydroxid, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (III) bzw. (Ia) in entsprechendem Überschuß gleichzeitig als Säurebindemittel einzusetzen.

Die Reaktionstemperaturen können bei der Durchführung der erfindungsgemäßen Verfahren (a) und (b) in einem größeren Bereich variiert werden. Im allgemeinen arbeitet man bei Temperaturen zwischen −20°C und +180°C, vorzugsweise bei Temperaturen zwischen 20°C und +150°C.

Zur Durchführung des erfindungsgemäßen Verfahrens (a) setzt man pro Mol an substituiertem Tetrahydrofuran der Formel (II) im allgemeinen 1,0 bis 3,0 Mol, vorzugsweise 1,0 bis 1,5 Mol an Amin der Formel (III) und gegebenenfalls 1,0 bis 3,0 Mol, vorzugsweise 1,0 bis 1,5 Mol an Säurebindemittel, sowie gegebenenfalls 0,1 bis 1,0 Mol an Phasentransferkatalysator ein.

Zur Durchführung des erfindungsgemäßen Verfahrens (b) setzt man pro Mol an Aminomethyltetrahydrofuran der Formel (Ia) im allgemeinen 1,0 bis 5,0 Mol, vorzugsweise 1,0 bis 2,0 Mol an Alkylierungsmittel der Formel (V) und 1,0 bis 5,0 Mol, vorzugsweise 1,0 bis 2,0 Mol an Säurebindemittel, sowie gegebenenfalls 0,1 bis 1,0 Mol an Phasentransferkatalysator ein.

Die Reaktionsdurchführung, Aufarbeitung und Isolierung der Reaktionsprodukte der Formel (I) erfolgt in beiden Fällen nach üblichen Methoden.

Zur Herstellung von Säureadditionssalzen der Verbindungen der Formel (I) kommen vorzugsweise folgende Säuren infrage : Halogenwasserstoffsäuren, wie z.B, Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p- Toluolsulfonsäure und 1,5-Naphthalindisulfonsäure sowie Saccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B, durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch u.a. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt :

Pythium-Arten, wie beispielsweise Pythium ultimum ; Phytophthora-Arten, wie beispielsweise Phytophthora infestans ;

Pseudoperonospora-Arten, wie beispielsweise Pseudoperonospora humuli oder Pseudoperonospora cubensis ;

Plasmopara-Arten, wie beispielsweise Plasmopara viticola ;

Peronospora-Arten, wie beispielsweise Peronospora pisi oder P, brassicae ;

Erysiphe-Arten, wie beispielsweise Erysiphe graminis ; Sphaerotheca-Arten, wie beispielsweise Sphaerotheca fuliginea ;

Podosphaera-Arten, wie beispielsweise Podosphaera leucotricha ;

Venturia-Arten, wie beispielsweise Venturia inaequalis ; Pyrenophora-Arten, wie beispielsweise Pyrenophora teres oder P. graminea

(Konidienform : Drechslera, Syn : Helminthosporium) ; CochlioboLus-Arten, wie beispielsweise Cochliobolus sativus

(Konidienform : Drechslera, Syn : Helminthosporium) ; Uromyces-Arten, wie beispielsweise Uromyces appendiculatus ;

Puccinia-Arten, wie beispielsweise Puccinia recondita ;

Tilletia-Arten, wie beispielsweise Tilletia caries ;

Ustilago-Arten, wie beispielsweise Ustilago nuda oder Ustilago avenae ;

Pellicularia-Arten, wie beispielsweise Pellicularia sasakii ;

Pyricularia-Arten, wie beispielsweise Pyricularia oryzae ;

Fusarium-Arten, wie beispielsweise Fusarium culmorum ;

Botrytis-Arten, wie beispielsweise Botrytis cinerea ;

Septoria-Arten, wie beispielsweise Septoria nodorum ;

Leptosphaeria-Arten, wie beispielsweise Leptosphaeria nodorum ;

Cercospora-Arten, wie beispielsweise Cercospora canescens ;

Alternaria-Arten, wie beispielsweise Alternaria brassicae ;

Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von

Getreidekrankheiten, wie beispielsweise gegen den Erreger des echten Gerstenmehltaus (Erysiphe graminis) oder gegen den Erreger der Braunfleckigkeit des Weizens (Leptosphaeria nodorum), zur Bekämpfung von Reiskrankheiten wie beispielsweise gegen den Erreger der Blattfleckenkrankheit am Reis (Pyricularia oryzae) oder zur Bekämpfung von Krankheiten im Obst- und Gemüseanbau wie beispielsweise gegen den Erreger des Apfelschorfes (Venturia inaequalis) einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen Für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier und/oder schaumerzeugende Mittel kommen in Frage : z.B. nichtionogene und anionische Emulgatoren, wie Polyoxyethylen-Fettsäure-Ester, Polyoxyethylen-Fettalkohol-Ether, z.B. Alkylarylpolyglykol- Ether, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 une 95 Gewichsprozent Wirkstoff, vorzugsweise swischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möghlich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorsugsweise swischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew,-%, vorzugsweise von 0,0001 bis 0,02% am Wirkungsort erforderlich.

Herstellunqsbeispiele :

Beispiel 1 :

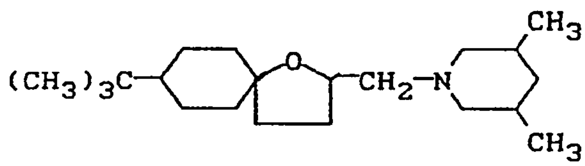

(Verfahren a)

Eine Mischung aus 15 g (0,05 Mol) 2-Brommethyl-8-t-butyl-1-oxaspiro[4,5]decan und 13 g (0,11 Mol) 3,5-Dimethylpiperidin wird 16 Stunden bei 140°C Badtemperatur gerührt, abgekühlt und in einer Mischung aus Diethylether und Wasser aufgenommen ; die organische Phase wird abgetrennt, über Natriumsulfat getrocknet, im Vakuum eingeengt und der Rückstand einer Kugelrohrdestillation unterworfen (Manteltemperatur 170°C bis 200°C bei 0,13 mbar).

Man erhält 9,5 g (57% der Theorie) an 8-t-Butyl-2-(3,5-dimethylpiperidin-1-yl-methyl)-1-oxaspiro[4,5]decan vom Brechungsindex $n_D^{20}$ 1.4869.

Herstellung der Ausgangsverbindung :

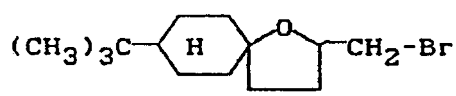

Zu einer Lösung von 42 g (0,2 Mol) 1-(3-Butenyl)-4-t-butyl-cyclohexanol in 600 ml absolutem Chloroform gibt man unter Rühren und Kühlung portionsweise 40 g (0,2 Mol) N-Bromsuccinimid, so daß die Temperatur der Reaktionsmischung 40°C nicht übersteigt. Nach beendeter Zugabe rührt man weitere 16 Stunden bei Raumtemperatur, wäscht zweimal mit Wasser, trocknet über Natriumsulfat und entfernt das Lösungsmittel im Vakuum.

Man erhält 43 g (74 % der Theorie) an 2-Brommethyl-8-t- butyl-1-oxaspiro[4,5]decan vom Brechungsindex $n_D^{20}$ = 1,4921.

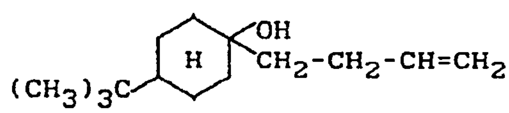

Zu einer Lösung aus 7,2 g (0,3 Mol) Magnesium und 40,5 g (0,3 Mol) 4-Brom-1-buten in 200 ml absolutem Diethylether tropft man bei Raumtemperatur unter Rühren eine Lösung von 45 g (0,3 Mol) 4-t-Butylcyclohexanon in 100 ml absolutem Tetrahydrofuran. Nach beendeter Zugabe rührt man 4 Stunden bei Rückflußtemperatur und hydrolysiert dann mit einer Mischung aus 2 normaler Chlorwasserstoffsäure und fis. Die organische Phase wird abgetrennt, über Natriumsulfat getrocknet und im Vakuum von Lösungsmittel befreit.

Nach Destillation im Vakuum erhält man 47 g (75% der Theorie) an 1-(3-Butenyl)-4-t-butyl-cyclohexanol vom Siedepunkt Kp 89°-91°C bei 0,1 mbar.

In entsprechender Weise und gemäß den allgemeinen Angaben zur Herstellung erhält man die folgenden Aminomethyltetrahydrofurane der allgemeinen Formel (I) :

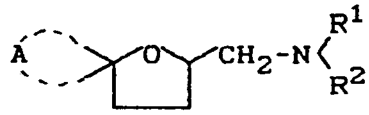

(I)

| Bsp. Nr. | 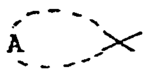 | 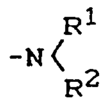 | physikalische Eigenschaften |
|---|---|---|---|
| 2 | 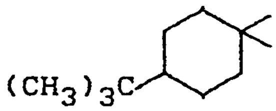 | 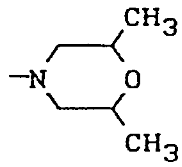 | $n_D^{20}$ 1,4878 |
| 3 | 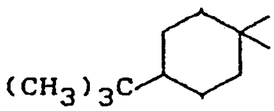 | 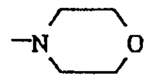 | $n_D^{20}$ 1,4928 |

| Bsp. Nr. | 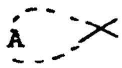 | 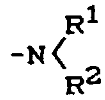 | physikalische Eigenschaften |
|---|---|---|---|
| 4 | 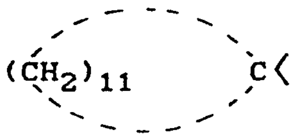 | 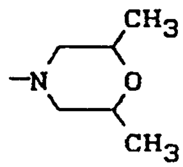 | $n_D^{20}$ 1,4939 |
| 5 | 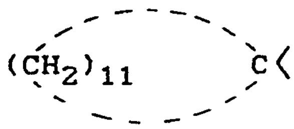 | 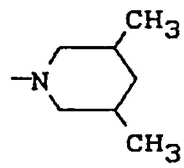 | $n_D^{20}$ 1,4983 |
| 6 | 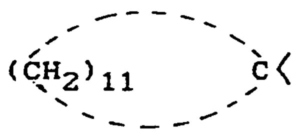 | 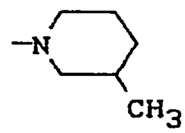 | $n_D^{20}$ 1,5002 |
| 7 | 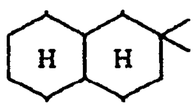 | 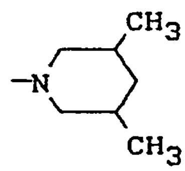 | $n_D^{20}$ 1,5190 |
| 8 | 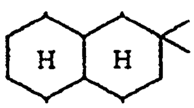 | 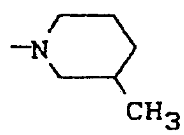 | $n_D^{20}$ 1,5025 |
| 9 | 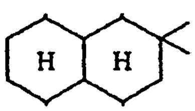 | 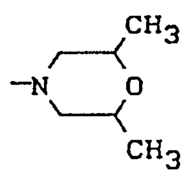 | $n_D^{20}$ 1,500 |
| 10 | 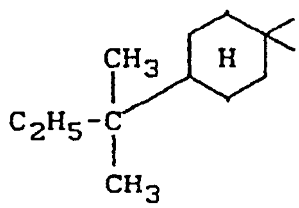 | $-NH-(CH_2)_2-CH_3$ | $n_D^{20}$ 1,4746 |

| Bsp. Nr. | 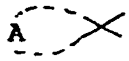 | 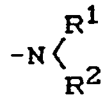 | physikalische Eigenschaften |
|---|---|---|---|
| 11 | 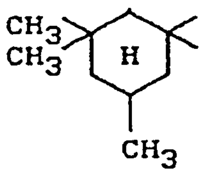 | 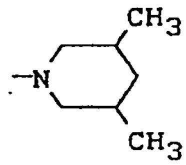 | $n_D^{20}$ 1,4768 |
| 12 | 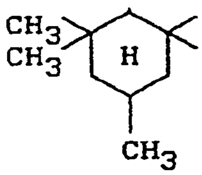 | 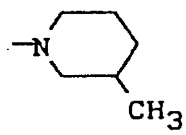 | $n_D^{20}$ 1,4759 |
| 13 | 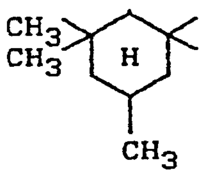 | 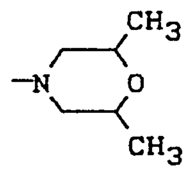 | $n_D^{20}$ 1,4713 |
| 14 | 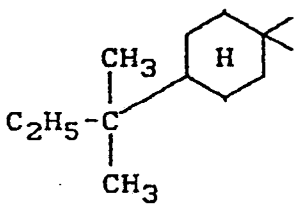 | 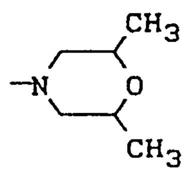 | $n_D^{20}$ 1,4831 |
| 15 | 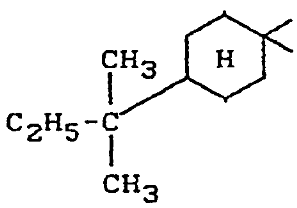 | 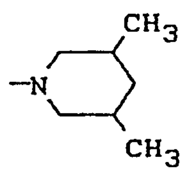 | $n_D^{20}$ 1,4830 |
| 16 | 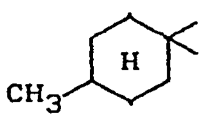 | 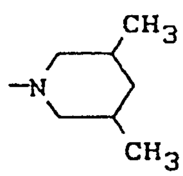 | $n_D^{20}$ 1,4786 |

| Bsp. Nr. | 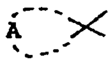 | 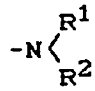 | physikalische Eigenschaften |
|---|---|---|---|
| 17 | 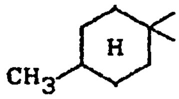 | 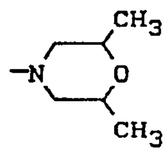 | $n_D^{20}$ 1,4800 |
| 18 | 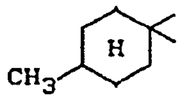 | 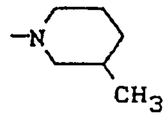 | $n_D^{20}$ 1,4860 |
| 19 | 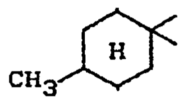 | 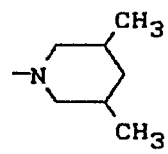 | $^1$H-NMR*)<br>4,35-4,5<br>3,45-3,9<br>2,8-2,95 |
| | | x 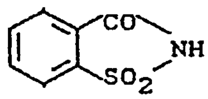 | |
| 20 | 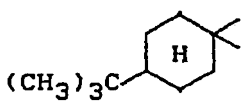 | 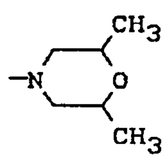 | Fp.39° -41° C |
| | | x 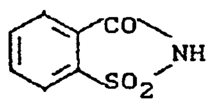 | |
| 21 | 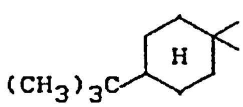 | 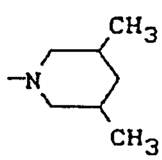 | Fp.37° -39° C |
| | | x 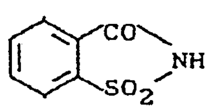 | |

| Bsp. Nr. | 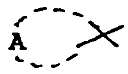 | $-N{<}^{R^1}_{R^2}$ | physikalische Eigenschaften |
|---|---|---|---|
| 22 | 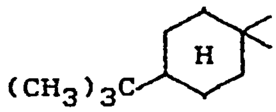 | $-NH-(CH_2)_3-O-C_2H_5$ | $n_D^{20}$ 1,4734 |
| 23 | 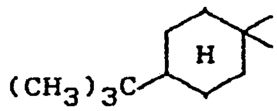 | $-NH-(CH_2)_3-O-C_2H_5$<br>x 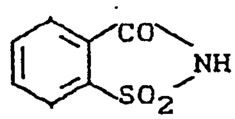 | amorph |
| 24 | 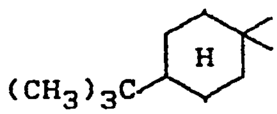 | $-NH-CH_2-CH(OCH_3)_2$ | $n_D^{20}$ 1,4610 |
| 25 | 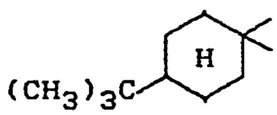 | $-NH-CH_2-CH(OC_2H_5)_2$ | $n_D^{20}$ 1,4650 |
| 26 | 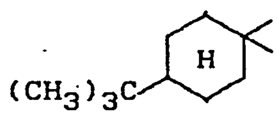 | $-NH-(CH_2)_3-O-(CH_2)_3-CH_3$ | $n_D^{20}$ 1,4700 |
| 27 | 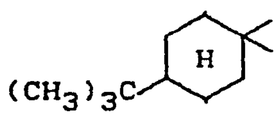 | 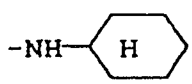 | $n_D^{20}$ 1,4864 |
| 28 | 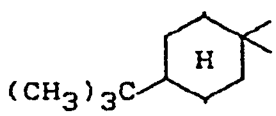 | $-NH-CH_2-CH(CH_3)_2$ | $n_D^{20}$ 1,4668 |
| 29 | 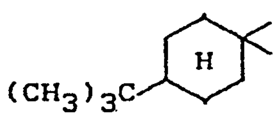 | $-NH-CH_2-CH(C_2H_5)_2$ | $n_D^{20}$ 1,4708 |

| Bsp. Nr. | 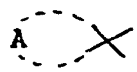 | 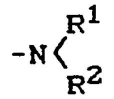 | physikalische Eigenschaften |
|---|---|---|---|
| 30 | 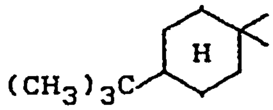 | $-NH-CH_2-CH(C_2H_5)_2$ x 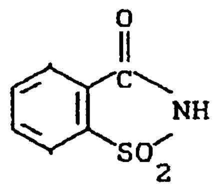 | Fp. -33° C |
| 31 | 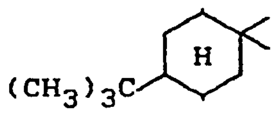 | 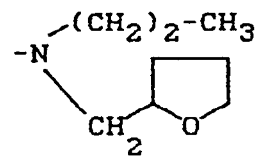 | $n_D^{20}$ 1,4784 |
| 32 | 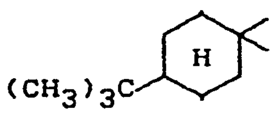 | 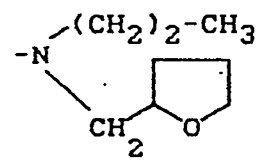 x 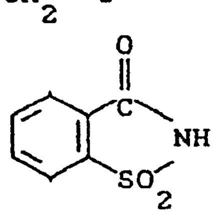 | Fp. -35° C |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

## Anwendungsbeispiele

In dem folgenden Anwendungbeispiel wurde die nachstehend aufgeführte Verbindung als Vergleichsubstanz eingesetzt :

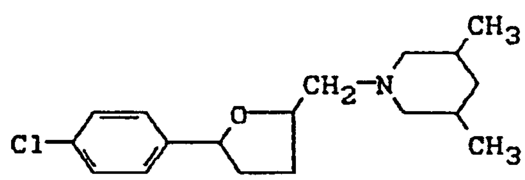

(A)

2-(4-Chlorphenyl)-5-(3,5-dimethylpiperidin-1-yl-methyl)-tetrahydrofuran (bekannt aus DE-OS 34 13 996).

## Beispiel A

Erysiphe-Test (Gerste) / protektiv
Lösungsmittel : 100 Gewichtsteile Dimethylformamid
Emulgator : 0,25 Gewichtsteile Alkylarylpolyglykolether
Zur Herstellung einer zweckmäßigen Wirkstoffzubereitung vermischt man 1 Gewichtsteil Wirkstoff mit den

angegebenen Mengen Lösungsmittel und Emulgator und verdünnt das Konzentrat mit Wasser auf die gewünschte Konzentration,

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen : 1, 2, 3, 4, 7, 8, 9, 10.

## Ansprüche

1. Aminomethyltetrahydrofurane der allgemeinen Formel (I),

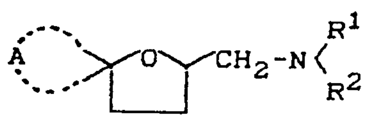

(I)

in welcher

A für eine jeweils gegebenenfalls einfach bis mehrfach, gleich oder verschieden substituierte zweifach verknüpfte Alkylen- oder Alkenylenkette mit jeweils 3 bis 18 Kohlenstoffatomen steht, wobei als Substituenten genannt seien :

jeweils geradkettiges oder verzweigtes Alkyl, Alkoxy, Alkylthio, Alkoxycarbonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einzelnen geradkettigen oder verzweigten Alkylteilen ; jeweils gegebenenfalls einfach bis mehrfach, gleich oder verschieden durch Alkyl mit 1 bis 4 Kohlenstoffatomen und Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil ;

jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes, jeweils zweifach verknüpftes Alkylen oder Alkenylen mit jeweils bis zu 5 Kohlenstoffatomen, jeweils gegebenenfalls einfach bis mehrfach, gleich oder verschieden substituiertes, einfach verknüpftes Aryl oder zweifach verknüpftes Arylen mit jeweils 6 bis 10 Kohlenstoffatomen, wobei als Substituenten genannt seien :

Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen,

$R^1$ und $R^2$ unabhängig voneinander jeweils für Wasserstoff ; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil stehen, wobei als Substituenten genannt seien :

Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen ; außerdem für jeweils gegebenenfalls einfach bis mehrfach, gleich oder verschieden im Arylteil substituiertes Aralkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten genannt seien :

Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den

einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom. enthalten kann, wobei als Substituenten genannt seien : jeweils geradkettiges oder verzweigtes Alkyl oder Hydroxyalkyl mit jeweils 1 bis 4 Kohlenstoffatomen,

deren Säureadditonssalze, deren geometrische und optische Isomere und/oder Isomerengemische.

2. Aminomethyltetrahydrofurane gemäß Anspruch 1, wobei in der Formel (I)

A für jeweils gegebenenfalls ein- bis fünffach, gleich oder verschieden substituiertes 1,4- Butylen, 1,5-Pentylen, 1,6-Hexylen, 1,5-Pent-1-enylen, 1,5-Pent-2-enylen oder 1,11-Unde-cylen steht, wobei als Substituenten jeweils genannt seien :

Methyl, Ethyl, n- oder i-Propyl, n-, i-, s- oder t-Butyl, n- oder i-Pentyl, n- oder i- Hexyl, n- oder i-Heptyl, n- oder i-Octyl, n- oder i-Nonyl, Methoxy, Ethoxy, n- oder i- Propoxy, n-, i-, s- oder t-Butoxy, Methylthio, Methoxycarbonyl, Ethoxycarbonyl, Trifluor-methyl, Trifluormethoxy, Trifluormethylthio, Trimethylsilyl, jeweils gegebenenfalls ein- bis dreifach, gleich oder verschieden durch Fluor, Chlor, Brom, Methyl, Ethyl, n- oder i- Propyl und/oder t-Butyl substituiertes Cyclohexyl, Cyclohexylmethyl, Cyclohexylethyl oder Cyclohexylpropyl, jeweils gegebenenfalls einbis dreifach, gleich oder verschieden durch Methyl, Ethyl, n- oder i-Propyl, n-, i-, s- oder t-Butyl, Trifluormethyl und Trifluormethoxy substituiertes Methylen, 1,2-Ethylen, 1,3-Propylen, 1,4-Butylen oder 1,4-Butenylen, oder jeweils gegebenenfalls ein- bis dreifach, gleich oder verschieden substituiertes Phenyl oder o-Phenylen, wobei als Substituenten im Phenyl oder o-Phenylen jeweils infrage kommen ; Fluor, Chlor, Brom, Cyano, Nitro, Methyl, Ethyl, n- oder i-Propyl, n-, i-, s- oder t- Butyl, Methoxy, Ethoxy, Trifluormethyl, Trifluormethoxy, Trifluormethylthio oder Methoximinomethyl und

$R^1$ und $R^2$ unabhängig voneinander jeweils für Wasserstoff, Methyl, Ethyl, n- oder i- Propyl, n-, i-, s- oder t-Butyl, n- oder i-Pentyl, n- oder i-Mexyl, n- oder i- Meptyl, n- oder i-Octyl, Allyl, n- oder i-Butenyl, n- oder i-Pentenyl, Propargyl, n- oder i-Butinyl, Hydroxyethyl, Hydroxypropyl, Methoxyethyl, Ethoxyethyl, Propoxyethyl, Butoxyethyl, Methoxypropyl, Ethoxypropyl, Propoxypropyl, Butoxypropyl, Hydroxyethoxyethyl, Dimethoxyethyl, Dimethoxypropyl, Diethoxyethyl, Dioxolanylmethyl, Dioxolanylethyl, Oxolanylmethyl, Oxolanylethyl, Dioxanylmethyl, Dioxanylethyl, für jeweils gegebenenfalls ein- bis fünffach, gleich oder verschieden durch Fluor, Chlor, Brom, Methyl, Ethyl, n- oder i-Propyl, n-, i-, s- und/oder t-Butyl substituiertes Cyclopropyl, Cyclopropylmethyl, Cyclopropylethyl, Cyclopropylpropyl, Cyclopentyl, Cyclopentylmethyl, Cyclohexyl oder Cyclohexylmethyl oder für jeweils gegebenenfalls ein- bis dreifach, gleich oder verschieden substituiertes Phenyl, Benzyl oder Phenylethyl stehen, wobei als Substituenten jeweils infrage kommen : Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Methoxycarbonyl, Ethoxycarbonyl oder Methoximinomethyl oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

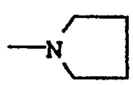 ; 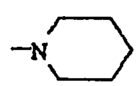 ; 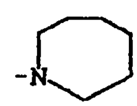 ; 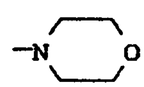

oder 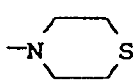

stehen, wobei als Substituenten jeweils genannt seien : Methyl, Ethyl oder Hydroxymethyl, deren Säureadditionssalze, deren geometrische und optischen Isomeren und/oder Isomerengemische.

3. Aminomethyltetrahydrofurane gemäß Anspruch 1, wobei in der Formel (I)

A gemeinsam mit dem Kohlenstoffatom, an welches es gebunden ist, für ein gegebenenfalls einbis dreifach, gleich oder verschieden substituiertes carbocyclisches Ringsystem der Formel

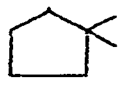 ; 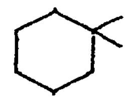 ; 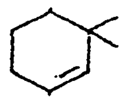 ; 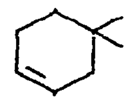 ; 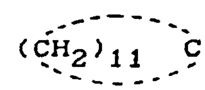

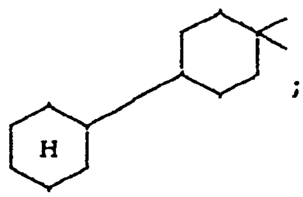 ; 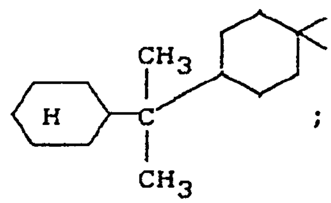 ; 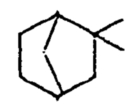 ;

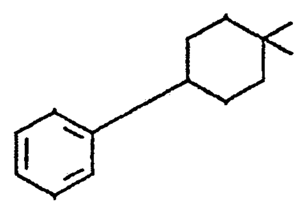 ; 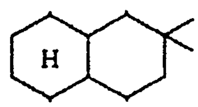 ; 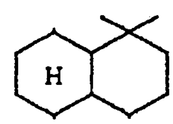

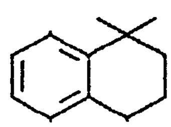 ; 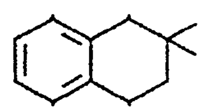 ; 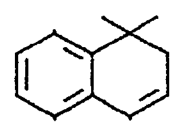 oder

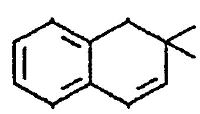 steht,

wobei als Substituenten in den cycloaliphatischen Ringen jeweils genannt seien : Methyl, Ethyl, n-oder i-Propyl, n-, i-, s- oder t- Butyl, n- oder i-Pentyl, Trifluormethyl, Trifluormethoxy, Methoxycarbonyl, Ethoxycarbonyl oder Trimethylsilyl und wobei als Substituenten in den aromatischen Ringen jeweils genannt seien : Fluor, Chlor, Brom, Methyl, Ethyl, n- oder i-Propyl, n-, i-, s- oder t-Butyl, Methoxy, Ethoxy, Trifluormethyl, Trifluormethoxy oder Trifluormethylthio und

$R^1$ und $R^2$ unabhängig voneinander jeweils für Wasserstoff, Methyl, Ethyl, n- oder i- Propyl, n-, i-, s- oder t-Butyl, n- oder i-Pentyl, n-oder i-Hexyl, Allyl, n- oder i-Butenyl, n- oder i-Pentenyl, Propargyl, n- oder i-Butinyl, Hydroxyethyl, Hydroxypropyl, Methoxyethyl, Methoxypropyl, Ethoxyethyl, Ethoxypropyl, Hydroxyethoxyethyl, Dimethoxyethyl, Diethoxyethyl, Dioxolanylmethyl, Dioxolanylethyl, Oxolanylmethyl, Oxolanylethyl, Dioxanylmethyl, Cyclopropylmethyl, Dichlorcyclopropylmethyl, Dimethylcyclopropylmethyl, Dichlordimethylcyclopropylmethyl, Cyclopentyl, Cyclohexyl oder Cyclohexylmethyl stehen oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

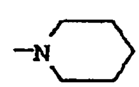 ; 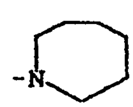 ; 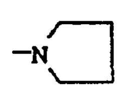 oder 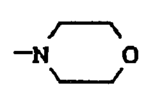

stehen,

wobei als Substituenten jeweils genannt seien :

Methyl, Ethyl, Hydroxymethyl, deren Säureadditionssalze, geometrische und optische Isomere und/oder Isomerengemische.

4. Verfahren zur Herstellung von Aminomethyltetrahydrofuranen der allgemeinen Formel (I),

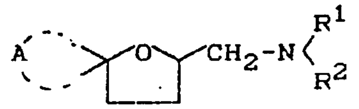

(I)

in welcher

A für eine jeweils gegebenenfalls einfach bis mehrfach, gleich oder verschieden substituierte zweifach verknüpfte Alkylen- oder Alkenylenkette mit jeweils 3 bis 18 Kohlenstoffatomen steht, wobei als Substituenten genannt seien :

jeweils geradkettiges oder verzweigtes Alkyl, Alkoxy, Alkylthio, Alkoxycarbonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einzelnen geradkettigen oder verzweigten Alkylteilen ; jeweils gegebenenfalls einfach bis mehrfach, gleich oder verschieden durch Alkyl mit 1 bis 4 Kohlenstoffatomen und Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil ; jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes, jeweils zweifach verknüpftes Alkylen oder Alkenylen mit jeweils bis zu 5 Kohlenstoffatomen, jeweils gegebenenfalls einfach bis mehrfach, gleich oder verschieden substituiertes, einfach verknüpftes Aryl oder zweifach verknüpftes Arylen mit jeweils 6 bis 10 Kohlenstoffatomen, wobei als Substituenten genannt seien ; Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen,

$R^1$ und $R^2$ unabhängig voneinander jeweils für Wasserstoff ; für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit jeweils 1 bis 6 Kohlenstoffatomen oder Hydroxyalkoxyalkyl mit 2 bis 6 Kohlenstoffatomen in den einzelnen Alkylteilen, für jeweils geradkettiges oder verzweigtes Dioxolanylalkyl, Oxolanylalkyl oder Dioxanylalkyl mit jeweils 1 bis 4 Kohlenstoffatomen im Alkylteil oder für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil stehen, wobei als Substituenten genannt seien : Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen ; außerdem für jeweils gegebenenfalls einfach bis mehrfach, gleich oder verschieden im Arylteil substituiertes Aralkyl, Arylalkenyl oder Aryl mit jeweils 6 bis 10 Kohlenstoffatomen im Arylteil und gegebenenfalls bis zu 6 Kohlenstoffatomen im geradkettigen oder verzweigten Alkyl- bzw. Alkenylteil stehen, wobei als Arylsubstituenten genannt seien :

Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach bis mehrfach, gleich oder verschieden substituierten, gesättigten 5- bis 7-gliedrigen Heterocyclus stehen, der gegebenenfalls ein weiteres Heteroatom enthalten kann, wobei als Substituenten genannt seien : jeweils geradkettiges oder verzweigtes Alkyl oder Hydroxyalkyl mit jeweils 1 bis 4 Kohlenstoffatomen,

sowie deren Säureadditionssalze, deren geometrische oder optische Isomere und deren Isomerengemische, dadurch gekennzeichnet, daß man

(a) substituierte Tetrahydrofurane der Formel (II),

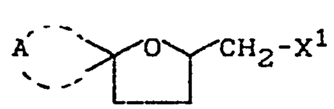

(II)

in welcher

A die oben angegebene Bedeutung hat und

$X^1$ für eine elektronenanziehende Abgangsgruppe steht, mit Aminen der Formel (III),

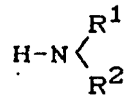

(III)

in welcher

$R^1$ und $R^2$ die oben angegebene Bedeutung haben, gegebenenfalls in Gegenwart eines Verdünnungsmittel und gegebenenfalls in Gegenwart eines Säurebindemitttels umsetzt, oder daß man

(b) die nach Verfahren (a) erhältlichen Aminomethyltetrahydrofurane der Formel (Ia),

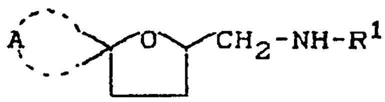

(Ia)

in welcher

$R^1$ und A die oben angegebene Bedeutung haben, mit Alkylierungsmitteln der Formel (V),

$$R^{2-1} - X^2 \quad (V)$$

in welcher

$R^{2-1}$ für jeweils geradkettiges oder verzweigtes Alkyl mit 1 bis 12 Kohlenstoffatomen, Alkenyl mit 3 bis 8 Kohlenstoffatomen, Alkinyl mit 3 bis 8 Kohlenstoffatomen, Hydroxyalkyl mit 2 bis 6 Kohlenstoffatomen, Alkoxyalkyl oder Dialkoxyalkyl mit 1 bis 6 Kohlenstoffatomen oder Hydroxyalkoxyalkyl mit 2 bis 6 Kohlenstoffatomen in den einzelnen Alkylteilen ; für jeweils geradkettiges oder verzweigtes Dioxolanylalkyl, Oxolanylalkyl oder Dioxanylalkyl mit jeweils 1 bis 4 Kohlenstoffatomen im Alkylteil ; für jeweils gegebenenfalls im Cycloalkylteil einfach bis mehrfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten jeweils infrage kommen ; Halogen, jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen ; außerdem für jeweils gegebenenfalls einfach bis mehrfach, gleich oder verschieden im Arylteil substituiertes Arylalkyl oder Arylalkenyl mit jeweils 6 bis 10 Kohlenstoffatomen im Arylteil und bis zu 6 Kohlenstoffatomen im geradkettigen oder verzweigten Alkyl-bzw. Alkenylteil steht, wobei als Arylsubstituenten jeweils infrage kommmen : Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls t bis 9 gleichen oder verschiedenen Halogenatomen und

$X^2$ für eine elektronenanziehende Abgangsgruppe steht,

gegebenenfalls in Gegenwart eines Verdünnungsmittels und gegebenenfalls in Gegenwart eines Säurebindemittels umsetzt und gegebenenfalls anschließend eine Säure addiert.

5. Schädlingsbekämpfungsmittel, gekennzeichnet durch einen Gehalt an mindestens einem Aminomethyltetrahydrofuran der Formel (I) nach den Ansprüchen 1 und 4.

6. Verwendung von Aminomethyltetrahydrofuranen der Formel (I) nach den Ansprüchen 1 und 4 zur Bekämpfung von Schädlingen.

7. Verfahren zur Bekämpfung von Schädlingen, dadurch gekennzeichnet, daß man Aminomethyltetrahydrofurane der Formel (I) nach den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man Aminomethyltetrahydrofurane der Formel (I) nach den Ansprüchen 1 und 4 mit Streckmitteln und/oder oberflächenaktiven Mitteln vermischt.

9. Substituierte Tetrahydrofurane der Formel (IIa),

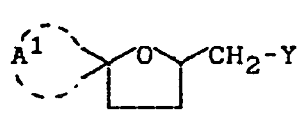

(IIa)

in welcher

$A^1$ für eine jeweils gegebenenfalls einfach bis mehrfach, gleich oder verschieden substituierte, zweifach

verknüpfte Alkylen- oder Alkenylenkette mit jeweils 3 bis 18 Kohlenstoffatomen steht, wobei als Substituenten genannt seien :

jeweils geradkettiges oder verzweigtes Alkyl, Alkoxy, Alkylthio, Alkoxycarbonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einzelnen geradkettigen oder verzweigten Alkylteilen ; jeweils gegebenenfalls einfach bis mehrfach, gleich oder verschieden durch Alkyl mit 1 bis 4 Kohlenstoffatomen und Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil ; jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes, jeweils zweifach verknüpftes Alkylen oder Alkenylen mit jeweils bis zu 5 Kohlenstoffatomen, jeweils gegebenenfalls einfach bis mehrfach, gleich oder verschieden substituiertes, einfach verknüpftes Aryl oder zweifach verknüpftes Arylen mit jeweils 6 bis 10 Kohlenstoffatomen, wobei als Substituenten genannt seien ; Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoximinoalkyl mit jeweils 1 bis 4 Kohlenstoffatomen in den einzelnen Alkylteilen und gegebenenfalls 1 bis 9 gleichen oder verschiedenen Halogenatomen,

Y für Halogen oder für jeweils gegebenenfalls substituiertes Alkylsulfonyloxy oder Arylsulfonyloxy steht,

mit Ausnahme der Verbindungen, bei welchen $A^1$ für eine 1,4-Butandiylkette und Y gleichzeitig für Brom oder Iod steht.

10. Verfahrren zur Herstellung von substituierten Tetrahydrofuranen der Formel (IIa),

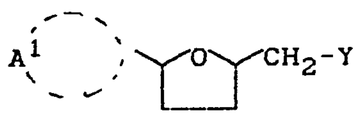

(IIa)

in welcher

$A^1$ für eine jeweils gegebenenfalls einfach bis mehrfach, gleich oder verschieden substituierte, zweifach verknüpfte Alkylen- oder Alkenylenkette mit jeweils 3 bis 18 Kohlenstoffatomen steht, wobei als Substituenten genannt seien :

jeweils geradkettiges oder verzweigtes Alkyl, Alkoxy, Alkylthio, Alkoxycarbonyl, Halogenalkyl, Halogenalkoxy oder Halogenalkylthio mit jeweils 1 bis 9 Kohlenstoffatomen und gegebenenfalls 1 bis 9 gleichen oder verschiedenen Halogenatomen ; Trialkylsilyl mit jeweils 1 bis 4 Kohlenstoffatomen in den einzelnen geradkettigen oder verzweigten Alkylteilen ; jeweils gegebenenfalls einfach bis mehrfach, gleich oder verschieden durch Alkyl mit 1 bis 4 Kohlenstoffatomen und Halogen substituiertes Cycloalkylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil ; jeweils gegebenenfalls einfach bis mehrfach, gleich oder verschieden durch Alkyl, Halogenalkyl, Alkoxy oder Halogenalkoxy mit jeweils 1 bis 4 Kohlenstoffatomen und gegebenenfalls 1 bis 9 gleichen oder verschiedenen Halogenatomen und/oder Halogen substituiertes, jeweils zweifach verknüpftes Alkylen oder Alkenylen mit jeweils bis zu 5 Kohlenstoffatomen, jeweils gegebenenfalls einfach bis mehrfach, gleich oder verschieden substituiertes, einfach verknüpftes Aryl oder zweifach verknüpftes Arylen mit jeweils 6 bis 10 Kohlenstoffatomen, wobei als Substituenten genannt seien ; Halogen, Cyano, Nitro, jeweils geradkettiges oder verzweigtes Alkyl, Alkoxy, Alkylthio, Halogenalkyl, Halogenalkoxy, Halogenalkylthio, Alkoxycarbonyl oder Alkoxyminoalkyl mit jeweils 1 bis 4 Kohlenstoffatomen in den einzelnen Alkylteilen und gegebenenfalls 1 bis 9 gleichen oder verschiedenen Halogenatomen,

Y für Halogen oder für jeweils gegebenenfalls substituiertes Alkylsulfonyloxy oder Arylsulfonyloxy steht,

mit Ausnahme der Verbindungen, bei welchen $A^1$ für eine 1,4-Butandiylkette und Y gleichzeitig für Brom oder Iod steht,

dadurch gekennzeichnet, daß man die cyclischen Ketone der Formel (VI),

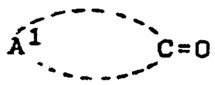

(VI)

in welcher

$A^1$ die oben angegebene Bedeutung hat, zunächst in einer ersten Stufe mit Buten-1-yl-4- magnesiumbromid der Formel (VII)

$$CH_2=CH-CH_2-CH_2-Mg-Br \quad (VII)$$

in Gegenwart eines Verdünnungsmittels bei Temperaturen zwischen −20°C und +50°C umsetzt und die so erhältlichen Carbinole der Formel (VIII),

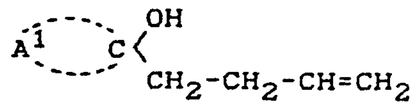

(VIII)

in welcher

$A^1$ die oben angegebene Bedeutung hat, mit einem Halogenierungsmittel, in Gegenwart von Chinolin oder mit N-Bromsuccinimid, gegebenenfalls in Gegenwart eines Verdünnungsmittels, bei Temperaturen zwischen −20°C und +80°C umsetzt, oder Trihydroxyverbindungen der Formel (IX),

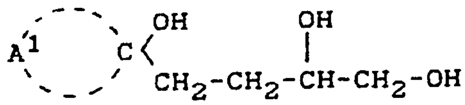

(IX)

in welcher

$A^1$ die oben angegebene Bedeutung hat, mit Säuren in üblicher Weise cyclisiert und die so erhältlichen Hydroxymethyltetrahydrofurane der Formel (X),

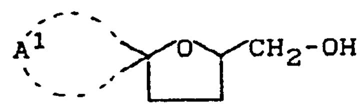

(X)

in welcher

$A^1$ die oben angegebene Bedeutung hat, in einer 2. Stufe mit Sulfonsäurehalogeniden der Formel (XI),

$$R^3-SO_2-Hal \quad (XI)$$

in welcher

$R^3$ für gegebenenfalls durch Halogen substituiertes Alkyl oder gegebenenfalls durch Alkyl mit 1 bis 4 Kohlenstoffatomen substituiertes Aryl steht und

Hal für Halogen steht,

gegebenenfalls in Gegenwart eines Verdünnungsmittels und gegebenenfalls in Gegenwart eines Säurebindemittels bei Temperaturen zwischen 0° und 120°C umsetzt.

## Claims

1. Aminomethyltetrahydrofurans of the general formula (I)

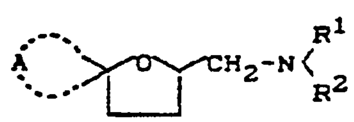

(I)

in which

A represents a divalent alkylene or alkenylene chain which has in each case 3 to 18 carbon atoms and is in each case optionally mono- or polysubstituted by identical or different substituents, substituents which may be mentioned being: in each case straight-chain or branched alkyl, alkoxy, alkylthio, alkoxycarbonyl, halogenoalkyl, halogenoalkoxy and halogenoalkylthio with in each case 1 to 9 carbon atoms and if appropriate 1 to 9 identical or different halogen atoms ; trialkylsilyl with in each case 1 to 4 carbon atoms in the individual straight-chain or branched alkyl parts ; cycloalkylalkyl or cycloalkyl which has in each case 3 to 7 carbon atoms

in the cycloalkyl part and if appropriate 1 to 4 carbon atoms in the straight-chain or branched alkyl part and is in each care optionally mono- or polysubstituted by identical or different subrtituents from the group comprising alkyl with 1 to 4 carbon atcms and halogen ; in each case divalent alkylene or alkenylene which has in each case up to 5 carbon atoms and is in each case optionally mono- or polysubstituted by identical or different substituents from the group comprising alkyl, halogenoalkyl, alkoxy and halogenoalkoxy with in each case 1 to 4 carbon atoms and if appropriate 1 to 9 identical or different halogen atoms and/or halogen, and monovalent aryl or divalent arylene which has in each case 6 to 10 carbon atoms and is in each case optionally mono- or polysubstituted by identical or different substituents, substituents which may be mentioned being : halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms,

$R^1$ and $R^2$ independently of one another each represent hydrogen ; or represent in each case straightchain or branched alkyl with 1 to 12 carbon atoms, alkenyl with 3 to 8 carbon atoms, alkinyl with 3 to 8 carbon atoms, hydroxyalkyl with 2 to 6 carbon atoms, alkoxyalkyl or dialkoxyalkyl with in each case 1 to 6 carbon atoms or hydroxyalkoxyalkyl with 2 to 6 carbon atoms in the individual alkyl parts, or represent in each case straight-chain or branched dioxolanylalkyl, oxolanylalkyl or dioxanylalkyl with in each case 1 to 4 carbon atoms in the alkyl part, or represent cycloalkyl or cycloalkylalkyl which has in each case 3 to 7 carbon atoms in the cycloalkyl part and if appropriate 1 to 4 carbon atoms in the alkyl part and is in each case optionally mono- or polysubstituted by identical or different substituents in the cycloalkyl part, substituents which may be mentioned being: halogen and in each case straight-chain or branched alkyl, alkoxy, halogenoalkyl and halogenoalkoxy with in each case 1 to 4 carbon atoms and if appropriate 1 to 9 identical or different halogen atoms ; or furthermore represent aralkyl, arylalkenyl or aryl which has in each case 6 to 10 carbon atoms in the aryl part and if appropriate up to 6 carbon atoms in the straight-chain or branched alkyl or alkenyl part and is in each case optionally mono- or polysubstituted by identical or different substituents in the aryl part, substituents on the aryl which may be mentioned being : halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms, or

$R^1$ and $R^2$, together with the nitrogen atom to which they are bonded, represent a saturated 5- to 7-membered heterocyclic radical which is optionally mono- or polysubstituted by identical or different substituents and can optionally contain a further heteroatom, substituents which may be mentioned being : in each case straight-chain or branched alkyl and hydroxyalkyl with in each case 1 to 4 carbon atoms,

acid addition salts thereof and geometric and optical isomers and/or isomer mixtures thereof.

2. Aminomethyltetrahydrofurans according to Claim 1, wherein, in formula (I),

A represents 1,4-butylene, 1,5-pentylene, 1,6- hexylene, 1,5-pent-1-enylene, 1,5-pent-2- enylene or 1,11-undecylene, in each case optionally mono-, di-, tri-, tetra- or pentasubstituted by identical or different substituents, substituents which may be mentioned in each case being : methyl, ethyl, n- or i- propyl, n-, i-, s- or t-butyl, n- or i-pentyl, n- or i-hexyl, n- or i-heptyl, n- or i-octyl, n- or i-nonyl, methoxy, ethoxy, n- or i-propoxy, n-, i-, s- or t-butoxy, methylthio, methoxycarbonyl, ethoxycarbonyl, trifluoromethyl, trifluoromethoxy, trifluoromethylthio and trimethylsilyl, cyclohexyl, cyclohexylmethyl, cyclohexylethyl and cyclohexylpropyl, in each case optionally mono-, di- or trisubatituted by identical or different substituents from the group comprising fluorine, chlorine, bromine, methyl, ethyl, n- or i-propyl and/or t-butyl ; methylene, 1,2-ethylene, 1,3-propylene, 1,4-butylene and 1,4-butenylene, in each case optionally mono-, di-or trisubstituted by identical or different substituents from the group comprising methyl, ethyl, n- or i-propyl, n-, i-, s- or t-butyl, trifluoromethyl and trifluoromethoxy, and phenyl and o-phenylene, in each case optionally mono-, di- or trisubstituted by identical or different substituents, possible substituents in the phenyl or o- phenylene in each case being : fluorine, chlorine, bromine, cyano, nitro, methyl, ethyl, n- or i-propyl, n-, i-, s- or t-butyl, methoxy, ethoxy, trifluoromethyl, trifluoromethoxy, trifluoromethylthio and methoximinomethyl, and

$R^1$ and $R^2$ independently of one another each represent hydrogen, methyl, ethyl, n- or i-propyl, n-, i-, s- or t-butyl, n- or i-pentyl, n- or i- hexyl, n- or i-heptyl, n- or i-octyl, allyl, n-or i-butenyl, n- or i-pentenyl, propargyl, n- or i-butinyl, hydroxyethyl, hydropropyl, methoxyethyl, ethoxyethyl, propoxyethyl, butoxyethyl, methoxypropyl, ethoxypropyl, propoxypropyl, butoxypropyl, hydroxyethoxyethyl, dimethoxyethyl, dimethoxypropyl, diethoxyethyl,dioxolanylmethyl, dioxolanylethyl, oxolanylmethyl, oxolanylethyl, dioxanylmethyl or dioxanylethyl, or represent cyclopropyl, cyclopropylmethyl, cyclopropylethyl, cyclopropylpropyl, cyclopentyl, cyclopentylmethyl, cyclohexyl or cyclohexylmethyl, in each case optionally mono-, di-, tri-, tetra- or pentasubstituted by identical or different substituents from the group comprising fluorine, chlorine, bromine, methyl, ethyl, n- or i-propyl and n-, i-, s- and/or t-butyl, or represent phenyl, benzyl or phenylethyl, in each case optionally mono-, di- or trisubstituted by identical or different substituents, possible substituents in each case being : fluorine, chlorine,

bromine, cyano, nitro, methyl, ethyl, n- or i-propyl, n-, i-, s- or t- butyl, methoxy, ethoxy, methylthio, trifluoromethyl, trifluoromethoxy, trifluoromethylthio, methoxycarbonyl, ethoxycarbonyl and methoximinomethyl, or

R[1] and R[2], together with the nitrogen atom to which they are bonded, represent a heterocyclic radical of the formula

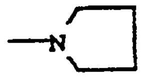 ; 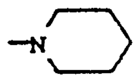 ; 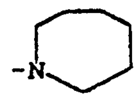 ; 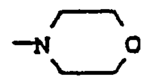

or

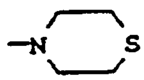

which is optionally mono-, di- or trisubstituted by identical or different substituents, substituents which may be mentioned being : methyl, ethyl and hydromethyl,
acid addition salts thereof and geometric and optical isomers and/or isomer mixtures thereof.

3. Aminomethyltetrahydrofurans according to Claim 1, wherein, in formula (I),

A together with the carbon atom to which it is bonded, represents a carbocyclic ring system of the formula

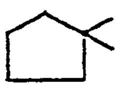 ; 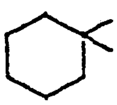 ; 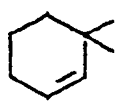 ; 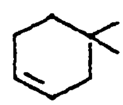 ; 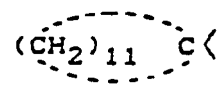

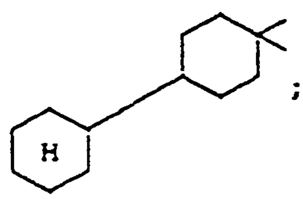 ; 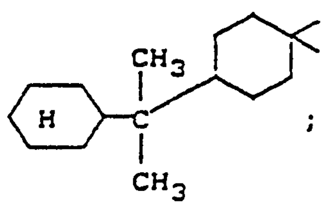 ; 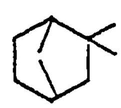 ;

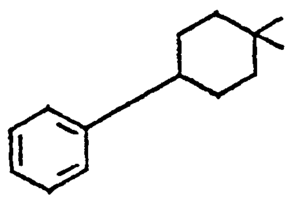 ; 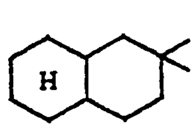 ; 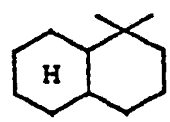

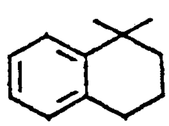 ; 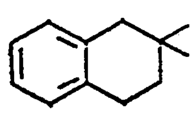 ; 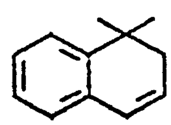 or

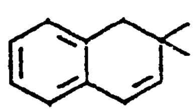

which is optionally mono-, di- or trisubstituted by identical or different substituents, substituents in the cycloaliphatic rings which may be mentioned in each case being : methyl, ethyl, n- or i-propyl, n-, i-, s- or t- butyl,

n- or i-pentyl, trifluoromethyl, trifluoromethoxy, methoxycarbonyl, ethoxycarbonyl and trimethylsilyl, and substituents in the aromatic rings which may be mentioned in each case being : fluorine, chlorine, bromine, methyl, ethyl, n- or i-propyl, n-, i-, s- or t-butyl, methoxy, ethoxy, trifluoromethyl, trifluoromethoxy and trifluoromethylthio, and

$R^1$ and $R^2$ independently of one another each represent hydrogen, methyl, ethyl, n- or i-propyl, n-, i-, s- or t-butyl, n- or i-pentyl, n- or i-hexyl, allyl, n- or i-butenyl, n- or i-pentenyl, propargyl, n- or i-butinyl, hydroxyethyl, hydroxypropyl, methoxyethyl, methoxypropyl, ethoxyethyl, ethoxypropyl, hydroxyethoxyethyl, dimethoxyethyl, diethoxyethyl, dioxolanylmethyl, dioxolanylethyl, oxolanylmethyl, oxolanylethyl, dioxanylmethyl, cyclopropylmethyl, dichlorocyclopropymethyl, dimethylcyclopropylmethyl, dichlorodimethylcyclopropylmethyl, cyclopentyl, cyclohexyl or cyclohexylmethyl, or

$R^1$ and $R^2$, together with the nitrogen atom to which they are bonded, represent a heterocyclic radical of the formula

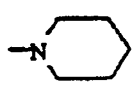 ; 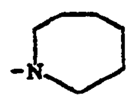 ; 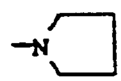 or 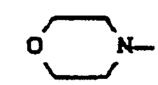

which is optionally mono-, di- or trisubstituted by identical or different substituents, substituents which may be mentioned in each case being : methyl, ethyl and hydroxymethyl,
acid addition salts thereof, geometric and optical isomers and/or isomer mixtures thereof.

4. Process for the preparation of aminomethyltetrahydrofurans of the general formula (I)

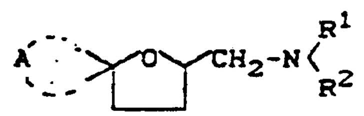

(I)

in which

A represents a divalent alkylene or alkenylene chain which has in each case 3 to 18 carbon atoms and is in each case optionally mono- or polysubstituted by identical or different substituents, substituents which may be mentioned being : in each case straight-chain or branched alkyl, alkoxy, alkylthio, alkoxycarbonyl, halogenoalkyl, halogenoalkoxy and halogenoalkylthio with in each case 1 to 9 carbon atoms and if appropriate 1 to 9 identical or different halogen atoms ; trialkylsilyl with in each case 1 to 4 carbon atoms in the individual straight-chain or branched alkyl parts ; cycloalkylalkyl or cycloalkyl which has in each case 3 to 7 carbon atoms in the cycloalkyl part and if appropriate 1 to 4 carbon atoms in the straight-chain or branched alkyl part and in in each case optionally mono- or polysubstituted by identical or different substituents from the group comprising alkyl with 1 to 4 carbon atoms and halogen ; in each case divalent alkylene or alkenylene which has in each case up to 5 carbon atoms and is in each case optionally mono- or polysubstituted by identical or different substituents from the group comprising alkyl, halogenoalkyl, alkoxy and halogenoalkoxy with in each case 1 to 4 carbon atoms and if appropriate 1 to 9 identical or different halogen atoms and/or halogen, and monovalent aryl or divalent arylene which has in each case 6 to 10 carbon atoms and is in each case optionally mono- or polysubstituted by identical or different substituents, substituents which may be mentioned being : halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms,

$R^1$ and $R^2$ independently of one another each represent hydrogen ; or represent in each case straight-chain or branched alkyl with 1 to 12 carbon atoms, alkenyl with 3 to 8 carbon atoms, alkinyl with 3 to 8 carbon atoms, hydroxyalkyl with 2 to 6 carbon atoms, alkoxyalkyl or dialkoxyalkyl with in each case 1 to 6 carbon atoms or hydroxyalkoxyalkyl with 2 to 6 carbon atoms in the individual alkyl parts, or represent in each case straight-chain or branched dioxolanylalkyl, oxolanylalkyl or dioxanylalkyl with in each case 1 to 4 carbon atoms in the alkyl part, or represent cycloalkyl or cycloalkylalkyl which has in each case 3 to 7 carbon atoms in the cycloalkyl part and if appropriate 1 to 4 carbon atoms in the alkyl part and is in each case optionally mono- or polysubstituted by identical or different substituents in the cycloalkyl part, substituents which may be mentioned being: halogen and in each case straight-chain or branched alkyl, alkoxy, halogenoalkyl and halogenoalkoxy with in each case 1 to 4 carbon atoms and if appropriate 1 to 9 identical or different halogen atoms ; or furthermore represent aralkyl, arylalkenyl or aryl which has in each case 6 to 10 carbon atoms in the aryl part and if appropriate up to 6 carbon atoms in the straight-chain or branched alkyl or alkenyl part and is in each case optionally

mono- or polysubstituted by identical or different substituents in the aryl part, substituents on the aryl which may be mentioned being : halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms, or

$R^1$ and $R^2$, together with the nitrogen atom to which they are bonded, represent a saturated 5- to 7-membered heterocyclic radical which is optionally mono- or polysubstituted by identical or different substituents and can optionally contain a further heteroatom, substituents which may be mentioned being : in each case straight-chain or branched alkyl and hydroxyalkyl with in each case 1 to 4 carbon atoms,

and acid addition salts thereof, geometric or optical isomers thereof and isomer mixtures thereof, characterized in that

(a) substituted tetrahydrofurans of the formula (II)

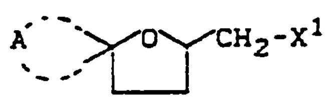 (II)

in which

A has the abovementioned meaning and

$X^1$ represents an electron-withdrawing leaving group, are reacted with amines of the formula (III)

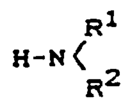 (III)

in which

$R^1$ and $R^2$ have the abovementioned meaning, if appropriate in the presence of a diluent and if appropriate in the presence of an acid-binding agent, or in that

(b) the aminomethyltetrahydrofurans obtainable by process (a), of the formula (Ia)

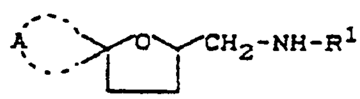 (Ia)

in which

$R^1$ and A have the abovementioned meaning, are reacted with alkylating agents of the formula (V)

$$R^{2-1} - X^2 \quad (V)$$

in which

$R^{2-1}$ represents in each case straight-chain or branched alkyl with 1 to 12 carbon atoms, alkenyl with 3 to 8 carbon atoms, alkinyl with 3 to 8 carbon atoms, hydroxyalkyl with 2 to 6 carbon atoms, alkoxyalkyl or dialkoxyalkyl with 1 to 6 carbon atoms or hydroxyalkoxyalkyl with 2 to 6 carbon atoms in the individual alkyl parts; or represents in each case straight-chain or branched dioxolanylalkyl, oxolanylalkyl or dioxanylalkyl with in each case 1 to 4 carbon atoms in the alkyl part ; or represents cycloalkyl or cycloalkylalkyl which has in each case 3 to 7 carbon atoms in the cycloalkyl part and if appropriate 1 to 4 carbon atoms in the alkyl part and is in each case optionally mono- or polysubstituted by identical or different substituents in the cycloalkyl part, possible substituents in each case being : halogen and in each case straightchain or branched alkyl, alkoxy, halogenoalkyl and halogenoalkoxy with in each case 1 to 4 carbon atoms and if appropriate 1 to 9 identical or different halogen atoms ; or furthermore represents arylalkyl or arylalkenyl which has in each case 6 to 10 carbon atoms in the aryl part and up to 6 carbon atoms in the straight-chain or branched alkyl or alkenyl part and is in each case optionally mono- or polysubstituted by identical or different substituents in the aryl part, possible substituents on the aryl in each case being : halogen, cyano, nitro, and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms and

$X^2$ represents an electron-withdrawing leaving group, if appropriate in the presence of a diluent and if appropriate in the presence of an acid-binding agent, and if appropriate an acid is then added on.

5. Agents for combating pests, characterized in that they contain at least one aminomethyltetrahydrofuran of the formula (I) according to Claims 1 and 4.

6. Use of aminomethyltetrahydrofurans of the formula (I) according to Claims 1 and 4 for combating pests.

7. Method of combating pests, characterized in that aminomethyltetrahydrofurans of the formula (I) according to Claims 1 and 4 are allowed to act on pests and/or their environment.

8. Process for the preparation of agents for combating pests, characterized in that aminomethyltetrahydrofurans of the formula (I) according to Claims 1 and 4 are mixed with extenders and/or surface-active agents.

9. Substituted tetrahydrofurans of the formula (IIa)

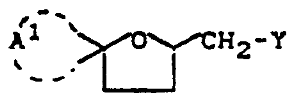

(IIa)

in which

$A^1$ represents a divalent alkylene or alkenylene chain which has in each case 3 to 18 carbon atoms and is in each case optionally mono- or polysubstituted by identical or different substituents, substituents which may be mentioned being: in each case straight-chain or branched alkyl, alkoxy, alkylthio, alkoxycarbonyl, halogenoalkyl, halogenoalkoxy and halogenoalkylthio with in each case 1 to 9 carbon atoms and if appropriate 1 to 9 identical or different halogen atoms; trialkylsilyl with in each case 1 to 4 carbon atoms in the individual straight-chain or branched alkyl parts; cycloalkylalkyl or cycloalkyl which has in each case 3 to 7 carbon atoms in the cycloalkyl part and if appropriate 1 to 4 carbon atoms in the straight-chain or branched alkyl part and is in each case optionally mono- or polysubstituted by identical or different substituents from the group comprising alkyl with 1 to 4 carbon atoms and halogen; in each case divalent alkylene or alkenylene which has in each case up to 5 carbon atoms and is in each case optionally mono- or polysubstituted by identical or different substituents from the group comprising alkyl, halogenoalkyl, alkoxy and halogenoalkoxy with in each case 1 to 4 carbon atoms and if appropriate 1 to 9 identical or different halogen atoms and/or halogen, and monovalent aryl or divalent arylene which has in each case 6 to 10 carbon atoms and is in each case optionally mono- or polysubstituted by identical or different substituents, substituents which may be mentioned being: halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms, and

Y represents halogen, or represents in each case optionally substituted alkylsulphonyloxy or arylsulphonyloxy,

with the exception of the compounds in which $A^1$ represents a 1,4-butanediyl chain and at the same time Y represents bromine or iodine.

10. Process for the preparation of substituted tetrahydrofurans of the formula (IIa)

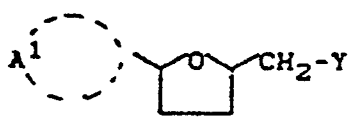

(IIa)

in which

$A^1$ represents a divalent alkylene or alkenylene chain which has in each case 3 to 18 carbon atoms and is in each case optionally mono- or polysubstituted by identical or different substituents, substituents which may be mentioned being: in each case straight-chain or branched alkyl, alkoxy, alkylthio, alkoxycarbonyl, halogenoalkyl, halogenoalkoxy and halogenoalkylthio with in each case 1 to 9 carbon atoms and if appropriate 1 to 9 identical or different halogen atoms; trialkylsilyl with in each case 1 to 4 carbon atoms in the individual straight-chain or branched alkyl parts; cycloalkylalkyl or cycloalkyl which has in each case 3 to 7 carbon atoms in the cycloalkyl part and if appropriate 1 to 4 carbon atoms in the straight-chain or branched alkyl part and is in each case optionally mono- or polysubstituted by identical or different substituents from the group comprising alkyl with 1 to 4 carbon atoms and halogen; in each case divalent alkylene or alkenylene which has in each case up to 5 carbon atoms and is in each case optionally mono- or polysubstituted by identical or different substituents from the group comprising alkyl, halogenoalkyl, alkoxyand halogenoalkoxy with in each case 1 to 4 carbon atoms and if appropriate 1 to 9 identical or different halogen atoms and/or halogen, and monovalent aryl or divalent arylene which has in each case 6 to 10 carbon atoms and is in each case optionally mono- or

polysubstituted by identical or different substituents, substituents which may be mentioned being : halogen, cyano, nitro and in each case straight-chain or branched alkyl, alkoxy, alkylthio, halogenoalkyl, halogenoalkoxy, halogenoalkylthio, alkoxycarbonyl and alkoximinoalkyl with in each case 1 to 4 carbon atoms in the individual alkyl parts and if appropriate 1 to 9 identical or different halogen atoms, and

Y represents halogen, or represents in each case optionally substituted alkylsulphonyloxy or arylsulphonyloxy,

with the exception of the compounds in which $A^1$ represents a 1,4-butanediyl chain and at the same time Y represents bromine or iodine,

characterized in that the cyclic ketones of the formula (VI)

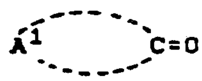

(VI)

in which

$A^1$ has the abovementioned meaning, are initially reacted in a first stage with buten-1-yl-4-magnesium bromide of the formula (VII)

$$CH_2=CH-CH_2-CH_2-Mg-Br \quad (VIII)$$

in the presence of a diluent at temperatures between −20°C and +50°C, and the carbinols thus obtainable, of the formula (VIII)

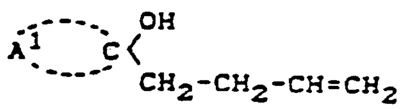

(VIII)

in which

$A^1$ has the abovementioned meaning, are reacted with a halogenating agent in the presence of quinoline or with N-bromosuccinimide, if appropriate in the presence of a diluent, at temperatures between −20°C and +80°C, or trihydroxy compounds of the formula (IX)

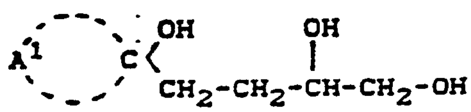

(IX)

in which

$A^1$ has the abovementioned meaning, are cyclized with acids in the customary manner and the hydroxymethyltetrahydrofurans thus obtainable, of the formula (X)

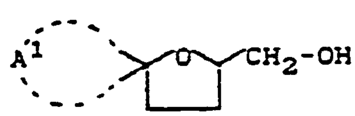

(X)

in which

$A^1$ has the abovementioned meaning, are reacted in a 2nd stage with sulphonic acid halides of the formula (XI)

$$R^3-SO_2-Hal \quad (XI)$$

in which

$R^3$ represents alkyl which is optionally substituted by halogen or aryl which is optionally substituted by alkyl with 1 to 4 carbon atoms and

Hal represents halogen,
if appropriate in the presence of a diluent and if appropriate in the presence of an acid-binding agent, at temperatures between 0° and 120°C.

## Revendications

1. Aminométhyltétrahydrofurannes de formule générale (I),

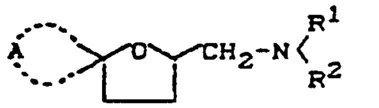

(I)

dans laquelle

A représente une chaîne alkylène ou une chaîne alcénylène à jonction double, ayant chacune 3 à 18 atomes de carbone et portant chacune le cas échéant un ou plusieurs substituants identiques ou différents, en considérant comme substituants :

un groupe alkyle, alkoxy, alkylthio, alkoxycarbonyle, halogénalkyle, halogénalkoxy ou halogénalkylthio chacun à chaîne droite ou ramifiée avec 1 à 9 atomes de carbone et, le cas échéant 1 à 9 atomes d'halogènes identiques ou différents ; un groupe trialkylsilyle ayant 1 à 4 atomes de carbone dans chacune des parties alkyle individuelles à chaîne droite ou ramifiée ; un groupe cycloalkylalkyle ou cycloalkyle ayant chacun 3 à 7 atomes de carbone dans la partie cycloalkyle et le cas échéant 1 à 4 atomes de carbone dans la partie alkyle à chaîne droite ou ramifiée, chacun portant éventuellement un ou plusieurs substituants, identiques ou différents, alkyle ayant 1 à 4 atomes de carbone et halogéno ; un groupe alkylène ou alcénylène ayant chacun 2 jonctions et jusqu'à 5 atomes de carbone et portant chacun le cas échéant un ou plusieurs substituants, identiques ou différents, alkyle, halogénalkyle, alkoxy ou halogénalkoxy ayant chacun 1 à 4 atomes de carbone et, le cas échéant, 1 à 9 atomes d'halogènes identiques ou différents, et/ou halogéno, un groupe aryle à une seule jonction ou un groupe arylène à deux jonctions ayant chacun 6 à 10 atomes de carbone et portant chacun le cas échéant un ou plusieurs substituants, identiques ou différents, en considérant comme substituants : un halogène, un radical cyano, nitro, alkyle, alkoxy, alkylthio, halogénalkyle, halogénalkoxy, halogénalkylthio, alkoxycarbonyle ou alkoxyiminoalkyle chacun à chaîne droite ou ramifiée avec, dans chaque cas, 1 a 4 atomes de carbone dans les parties alkyle individuelles et, le cas échéant, 1 à 9 atomes d'halogènes identiques ou différents et

$R^1$ et $R^2$ représentent chacun, indépendamment l'un de l'autre, l'hydrogène ; un groupe alkyle à chaîne droite ou ramifiée ayant 1 à 12 atomes de carbone, alcényle à chaîne droite ou ramifiée ayant 3 à 8 atomes de carbone, alcynyle à chaîne droite ou ramifiée ayant 3 à 8 atomes de carbone, hydroxyalkyle à chaîne droite ou ramifiée ayant 2 à 6 atomes de carbone, alkoxyalkyle ou dialkoxyalkyle à chaîne droite ou ramifiée ayant chacun 1 à 6 atomes de carbone ou hydroxyalkoxyalkyle ayant 2 à 6 atomes de carbone dans les parties alkyle individuelles, un groupe dioxolanylalkyle, oxolanylalkyle ou dioxanylalkyle chacun à chaîne droite ou ramifiée avec, dans chaque cas, 1 à 4 atomes de carbone dans la partie alkyle ou un groupe cycloalkyle ou cycloalkylalkyle ayant chacun 3 à 7 atomes de carbone dans la partie cycloalkyle et le cas échéant, 1 à 4 atomes de carbone dans la partie alkyle, et portant chacun le cas échéant dans la partie cycloalkyle un ou plusieurs substituants identiques ou différents, en considérant comme substituants :

un halogène, un groupe alkyle, alkoxy, halogénalkyle ou halogénalkoxy chacun à chaîne droite ou ramifiée avec dans chaque cas 1 à 4 atomes de carbone et, le cas échéant 1 à 9 atomes d'halogènes identiques ou différents ; en outre un groupe aralkyle, arylalcényle ou aryle ayant chacun 6 à 10 atomes de carbone dans la partie aryle et le cas échéant jusqu'à 6 atomes de carbone dans la partie alkyle ou alcényle à chaîne droite ou ramifiée et portant le cas échéant un ou plusieurs substituants identiques ou différents dans la partie aryle, en considérant comme substituants de la partie aryle : un halogène, un groupe cyano, nitro, un groupe alkyle, alkoxy, alkylthio, halogénalkyle, halogénalkoxy, halogénalkylthio, alkoxycarbonyle ou alkoxyiminoalkyle chacun à chaîne droite ou ramifiée avec, dans chaque cas, 1 à 4 atomes de carbone dans les parties alkyle individuelles et, le cas échéant, 1 à 9 atomes d'halogènes identiques ou différents, ou bien

$R^1$ et $R^2$ forment, conjointement avec l'atome d'azote auquel ils sont liés, un hétérocycle pentagonal à heptagonal saturé, portant le cas échéant un ou plusieurs substituants identiques ou différents et pouvant contenir éventuellement un autre hétéroatome, en considérant comme substituants : un groupe alkyle ou hydroxyalkyle chacun à chaîne droite ou à chaîne ramifiée, ayant, dans chaque cas, 1 à 4 atomes de carbone,

leurs sels d'addition d'acides, leurs isomères géométriques et optiques et/ou des mélanges d'isomères.

2. Aminométhyltétrahydrofurannes suivant la revendication 1, dans la formule (I) desquelles

A désigne un groupe 1,4-butylène, 1,5-pentylène, 1,6-hexylène, 1,5-pent-1-énylène, 1,5-pent-2- énylène ou 1,11-undécylène portant chacun le cas échéant 1 à 5 substituants identiques ou différents, en considérant comme substituants dans chaque cas :

un groupe méthyle, éthyle, n-propyle, isopropyle, n-butyle, isobutyle, sec.-butyle, tertiobutyle, n-pentyle, isopentyle, n-hexyle, isohexyle, n-heptyle, isoheptyle, n-octyle, iso-octyle, n-nonyle, isononyle, méthoxy, éthoxy, n-propoxy, isopropoxy, n-butoxy, isobutoxy, sec.-butoxy, tertiobutoxy, méthylthio, méthoxycarbonyle, éthoxycarbonyle, trifluorométhyle, trifluorométhoxy, trifluorométhylthio, triméthylsilyle, un groupe cyclohexyle, cyclohexylméthyle, cyclohexyléthyle ou cyclohexylpropyle portant chacun le cas échéant 1 à 3 substituants, identiques ou différents, fluoro, chloro, bromo, méthyle, éthyle, n-propyle, isopropyle et/ou tertiobutyle, un groupe méthylène, 1,2-éthylène, 1,3- propylène, 1,4-butylène ou 1,4-buténylène portant chacun le cas échéant 1 à 3 substituants, identiques ou différents, méthyle, éthyle, n-propyle, isopropyle, n-butyle, isobutyle, sec.-butyle, tertiobutyle, trifluorométhyle et trifluorométhoxy, ou un groupe phényle ou o-phénylène portant chacun le cas échéant 1 à 3 substituants identiques ou différents, en considérant comme substituants de chacun des groupes phényle ou o-phénylène : le fluor, le chlore, le brome, un groupe'cyano, nitro, méthyle, éthyle, n-propyle, isopropyle, n-butyle, isobutyle, sec.-butyle, tertiobutyle, méthoxy, éthoxy, trifluorométhyle, trifluorométhoxy, trifluorométhylthio ou méthoxyiminométhyle et

$R^1$ et $R^2$ représentent chacun, indépendamment l'un de l'autre, l'hydrogène, un groupe méthyle, éthyle, n-propyle, isopropyle, n-butyle, isobutyle, sec.-butyle, tertiobutyle, npentyle, isopentyle, n-hexyle, isohexyle, nheptyle, isoheptyle, n-octyle, iso-octyle, allyle, n-butényle, isobuténuyle, n-pentényle, isopentényle, propargyle, n-butinyle, isobutinyle, hydroxyéthyle, hydroxypropyle, méthoxyéthyle, éthoxyéthyle, propoxyéthyle, butoxyéthyle, méthoxypropyle, éthoxypropyle, propoxypropyle, butoxypropyle, hydroxyéthoxyéthyle, diméthoxyéthyle, diméthoxypropyle, diéthoxyéthyle, dioxolanylméthyle, dioxolanyléthyle, oxolanylméthyle, oxolanyléthyle, dioxanylméthyle, dioxanyléthyle, un groupe cyclopropyle, cyclopropylméthyle, cyclopropyléthyle, cyclopropylpropyle, cyclopentyle, cyclopentylméthyle, cyclohexyle ou cyclohexylméthyle portant chacun le cas échéant 1 à 5 substituants, identiques ou différents, fluoro, chloro, bromo, méthyle, éthyle, n-propyle, isopropyle, n-butyle, isobutyle, sec.-butyle et/ou tertiobutyle, ou un groupe phényle, benzyle ou phényléthyle portant chacun le cas échéant 1 à 3 substituants identiques ou différents, en considérant comme substituants dans chaque cas : le fluor, le chlore, le brome, un groupe cyano, nitro, méthyle, éthyle, n-propyle, isopropyle, n-butyle, isotiutyle, sec.-butyle, tertiobutyle, méthoxy, éthoxy, méthylthio, trifluorométhyle, trifluorométhoxy, trifluorométhylthio, méthoxycarbonyle, éthoxycarbonyle ou méthoxyiminométhyle, ou bien

$R^1$ et $R^2$ forment, conjointement avec l'atome d'azote auquel ils sont liés, un hétérocycle, portant éventuellement 1 à 3 substituants identiques ou différents, de formule

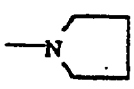 ; 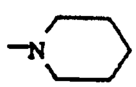 ; 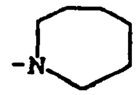 ; 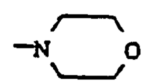

ou 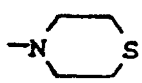

en considérant comme substituants dans chaque cas : un groupe méthyle, éthyle ou hydroxyméthyle, leurs sels d'addition d'acides, leurs isomères géométriques et optiques et/ou des mélanges d'isomères.

3. Aminométhyltétrahydrofurannes suivant la revendication 1, dans la formule (I) desquels

A forme conjointement avec l'atome de carbone auquel il est lié un noyau carbocyclique, portant éventuellement un à trois substituants identiques ou différents, de formule :

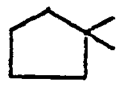 ; 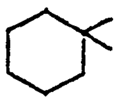 ; 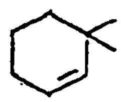 ; 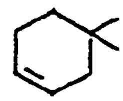 ; 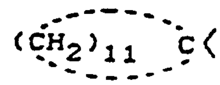

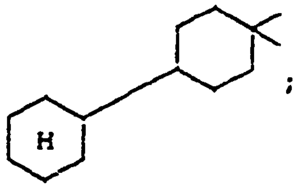 ; 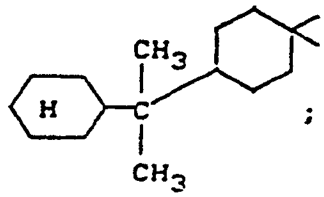 ; 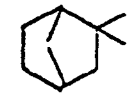 ;

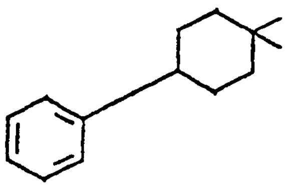 ; 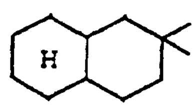 ; 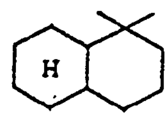

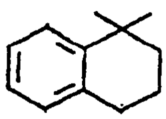 ; 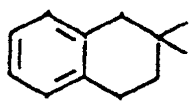 ; 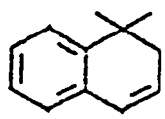 ou

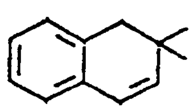

en considérant dans chaque cas comme substituants des noyaux cycloaliphatiques : un groupe méthyle, éthyle, n-propyle, isopropyle, n-butyle, isobutyle, sec.-butyle, tertiobutyle, n-pentyle, isopentyle, trifluorométhyle, trifluorométhoxy, méthoxycarbonyle, éthoxycarbonyle, triméthylsilyle et en considérant dans chaque cas comme substituants des noyaux aromatiques : le fluor, le chlore, le brome, un groupe méthyle, éthyle, n-propyle, isopropyle, n-butyle, isobutyle, sec.-butyle, tertiobutyle, méthoxy, éthoxy, trifluorométhyle, trifluorométhoxy ou trifluorométhylthio et

$R^1$ et $R^2$ représentent chacun, indépendamment l'un de l'autre, l'hydrogène, un groupe méthyle, éthyle, n-propyle, isopropyle, n-butyle, isobutyle, sec.-butyle, tertiobutyle, n- pentyle, isopentyle, n-hexyle, isohexyle, allyle, n-butényle, isobutényle, n-pentényle, isopentényle, propargyle, n-butinyle, isobutinyle, hydroxyéthyle, hydroxypropyle, méthoxyéthyle, méthoxypropyle, éthoxyéthyle, éthoxypropyle, hydroxyéthoxyéthyle, diméthoxyéthyle, diéthoxyéthyle, dioxolanylméthyle, dioxolanyléthyle, oxolanylméthyle, oxolanyléthyle, dioxanylméthyle, cyclopropylméthyle, dichlorocyclopropylméthyle, diméthylcyclopropylméthyle, dichlorodiméthylcyclopropylméthyle, cyclopentyle, cyclohexyle ou cyclohexylméthyle, ou bien

$R^1$ et $R^2$ forment, conjointement avec l'atome d'azote auquel ils sont liés, un hétérocycle, portant éventuellement 1 à 3 substituants identiques ou différents, de formule

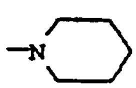 ; 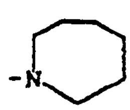 ; 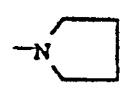 ou 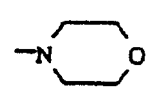

en considérant dans chaque cas comme substituants :

un groupe méthyle, éthyle, hydroxyméthyle, leurs sels d'addition d'acides, leurs isomères géométriques et optiques et/ou leurs mélanges.

4. Procédé de production d'aminométhyltétrahydrofurannes de formule générale (I)

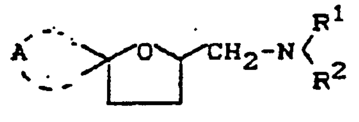

(I)

dans laquelle A représente une chaîne alkylène ou une chaîne alcénylène à jonction double, ayant chacune 3 à 18 atomes de carbone et portant chacune le cas échéant un ou plusieurs substituants identiques ou différents, en considérant comme substituants :

un groupe alkyle, alkoxy, alkylthio, alkoxycarbonyle, halogénalkyle, halogénalkoxy ou halogénalkylthio chacun à chaîne droite ou ramifiée avec 1 à 9 atomes de carbone et, le cas échéant 1 à 9 atomes d'halogènes identiques ou différents ; un groupe trialkylsilyle ayant 1 à 4 atomes de carbone dans chacune des parties alkyle individuelles à chaîne droite ou ramifiée ; un groupe cycloalkylalkyle ou cycloalkyle ayant chacun 3 à 7 atomes de carbone dans la partie cycloalkyle et le cas échéant 1 à 4 atomes de carbone dans la partie alkyle à chaîne droite ou ramifiée, chacun portant éventuellement 1 ou plusieurs substituants, identiques ou différents, alkyle ayant 1 à 4 atomes de carbone et halogéno ; un groupe alkylène ou alcénylène ayant chacun 2 jonctions et jusqu'à 5 atomes de carbone et portant chacun le cas échéant un ou plusieurs substituants, identiques ou différents, alkyle, halogénalkyle, alkoxy ou halogénalkoxy ayant chacun 1 à 4 atomes de carbone et, le cas échéant, 1 à 9 atomes d'halogènes identiques ou différents, et/ou halogéno, un groupe aryle à une seule jonction ou un groupe arylène à deux jonctions ayant chacun 6 à 10 atomes de carbone et portant chacun le cas échéant un ou plusieurs substituants, identiques ou différents, en considérant comme substituants : un halogène, un radical cyano, nitro, alkyle, alkoxy, alkylthio, halogénalkyle, halogénalkoxy, halogénalkylthio, alkoxycarbonyle ou alkoxyiminoalkyle chacun à chaîne droite ou ramifiée avec, dans chaque cas, 1 à 4 atomes de carbone dans les parties alkyle individuelles et, le cas échéant, 1 à 9 atomes d'halogènes identiques ou différents et

$R^1$ et $R^2$ représentent chacun, indépendamment l'un de l'autre, l'hydrogène ; un groupe alkyle à chaîne droite ou ramifiée ayant 1 à 12 atomes de carbone, alcényle à chaîne droite ou ramifiée ayant 3 à 8 atomes de carbone, alcynyle à chaîne droite ou ramifiée ayant 3 à 8 atomes de carbone, hydroxyalkyle à chaîne droite ou ramifiée ayant 2 à 6 atomes de carbone, alkoxyalkyle ou dialkoxyalkyle à chaîne droite ou ramifiée ayant chacun 1 à 6 atomes de carbone ou hydroxyalkoxyalkyle ayant 2 à 6 atomes de carbone dans les parties alkyle individuelles, un groupe dioxolanylalkyle, oxolanylalkyle ou dioxanylalkyle chacun à chaîne droite ou ramifiée avec, dans chaque cas, 1 à 4 atomes de carbone dans la partie alkyle ou un groupe cycloalkyle ou cycloalkylalkyle ayant chacun 3 à 7 atomes de carbone dans la partie cycloalkyle et le cas échéant, 1 à 4 atomes de carbone dans la partie alkyle, et portant chacun le cas échéant dans la partie cycloalkyle un ou plusieurs substituants identiques ou différents, en considérant comme substituants :

un halogène, un groupe alkyle, alkoxy, halogénalkyle ou halogénalkoxy chacun à chaîne droite ou ramifiée avec dans chaque cas 1 à 4 atomes de carbone et, le cas échéant 1 à 9 atomes d'halogènes identiques ou différents ; en outre un groupe aralkyle, arylalcényle ou aryle ayant chacun 6 à 10 atomes de carbone dans la partie aryle et le cas échéant jusqu'à 6 atomes de carbone dans la partie alkyle ou alcényle à chaîne droite ou ramifiée et portant le cas échéant un ou plusieurs substituants identiques ou différents dans la partie aryle, en considérant comme substituants de la partie aryle : un halogène, un groupe cyano, nitro, un groupe alkyle, alkoxy, alkylthio, halogénalkyle, halogénalkoxy, halogénalkylthio, alkoxycarbonyle ou alkoxyiminoalkyle chacun à chaîne droite ou ramifiée avec, dans chaque cas, 1 à 4 atomes de carbone dans les parties alkyle individuelles et, le cas échéant, 1 à 9 atomes d'halogènes identiques ou différents, ou bien

$R^1$ et $R^2$ forment, conjointement avec l'atome d'azote auquel ils sont liés, un hétérocycle pentagonal à heptagonal saturé, portant le cas échéant un ou plusieurs substituants identiques ou différents et pouvant contenir éventuellement un autre hétéroatome, en considérant comme substituants : un groupe alkyle ou hydroxyalkyle chacun à chaîne droite ou à chaîne ramifiée, ayant, dans chaque cas, 1 à 4 atomes de carbone, ainsi que de leurs sels d'addition d'acides, de leurs isomères géométriques ou optiques et de leurs mélanges d'isomères, caractérisé en ce que :

(a) on fait réagir des tétrahydrofurannes substitués de formule (II)

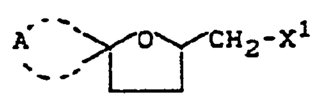

(II)

dans laquelle

A a la définition indiquée ci-dessus et

$X^1$ est un groupe partant attirant des électrons, avec des amines de formule (III)

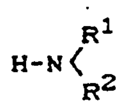 (III)

dans laquelle

$R^1$ et $R^2$ ont la définition indiquée ci-dessus, le cas échéant en présence d'un diluant et en la présence éventuelle d'un accepteur d'acide, ou bien

(b) on fait réagir les aminométhyltétrahydrofurannes obtenus par le procédé (a), de formule (Ia)

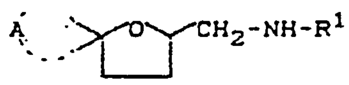 (Ia)

dans laquelle

$R^1$ et A ont la définition indiquée ci-dessus, avec des agents alkylants de formule (V),

$$R^{2-1} - X^2 \quad (V)$$

dans laquelle

$R^{2-1}$ est un groupe alkyle à chaîne droite ou ramifiée ayant 1 à 12 atomes de carbone, un groupe alcényle à chaîne droite ou ramifiée ayant 3 à 8 atomes de carbone, un groupe alcinyle à chaîne droite ou ramifiée ayant 3 à 8 atomes de carbone, un groupe hydroxyalkyle à chaîne droite ou ramifiée ayant 2 à 6 atomes de carbone, un groupe alkoxyalkyle ou dialkoxyalkyle à chaîne droite ou ramifiée ayant 1 à 6 atomes de carbone ou un groupe hydroxyalkoxyalkyle ayant 2 à 6 atomes de carbone dans les parties alkyle individuelles ; un groupe dioxolanylalkyle, oxolanylalkyle ou dioxanylalkyle ayant chacun une chaîne droite ou ramifiée avec 1 à 4 atomes de carbone dans la partie alkyle ; un groupe cycloalkyle ou cycloalkylalkyle ayant chacun 3 à 7 atomes de carbone dans la partie cycloalkyle et, le cas échéant 1 à 4 atomes de carbone dans la partie alkyle et portant chacun le cas échéant dans la partie cycloalkyle un ou plusieurs substituants identiques ou différents, en considérant dans chaque cas comme substituants : un halogène, un groupe alkyle, alkoxy, halogénalkyle ou halogénalkoxy à chaîne droite ou ramifiée ayant chacun 1 à 4 atomes de carbone et, le cas échéant, 1 à 9 atomes d'halogènes identiques ou différents ; en outre un groupe arylalkyle ou arylalcényle ayant chacun 6 à 10 atomes de carbone dans la partie aryle et jusqu'à 6 atomes de carbone dans la partie alkyle ou alcényle à chaîne droite ou à chaîne ramifiée, portant chacun le cas échéant un ou plusieurs substituants identiques ou différents dans la partie aryle, en considérant dans chaque cas comme substituants de la partie aryle : un halogène, un groupe cyano, nitro, un groupe alkyle, alkoxy, alkylthio, halogénalkyle, halogénalkoxy, halogénalkylthio, alkoxycarbonyle ou alkoxyiminoalkyle chacun à chaîne droite ou à chaîne ramifiée avec, dans chaque cas, 1 à 4 atomes de carbone dans les parties alkyle individuelles et, le cas échéant, 1 à 9 atomes d'halogènes identiques ou différents et

$X^2$ est un groupe partant attirant les électrons,

le cas échéant en présence d'un diluant et en la présence éventuelle d'un accepteur d'acide, et on additionne ensuite éventuellement un acide.

5. Composition pesticide, caractérisée par une teneur en au moins un aminométhyltétrahydrofuranne de formule (I) suivant les revendications 1 et 4.

6. Utilisation d'aminométhyltétrahydrofurannes de formule (I) suivant les revendications 1 et 4 pour combattre des parasites.

7. Procédé pour combattre des parasites, caractérisé en ce qu'on fait agir des aminométhyltétrahydrofurannes de formule (I) suivant les revendications 1 et 4 sur des parasites et/ou sur leur milieu.

8. Procédé de préparation de compositions destinées à lutter contre des parasites, caractérisé en ce qu'on mélange des aminométhyltétrahydrofurannes de formule (I) suivant les revendications 1 et 4 avec des diluants et/ou des agents tensio-actifs.

9. Tétrahydrofurannes substitués de formule (IIa)

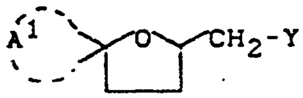

(IIa)

dans laquelle

A[1] désigne une chaîne alkylène ou une chaîne alcénylène à deux jonctions, ayant chacune 3 à 18 atomes de carbone et portant chacune le cas échéant un ou plusieurs substituants identiques ou différents, en considérant comme substituants :

un groupe alkyle, alkoxy, alkylthio, alkoxycarbonyle, halogénalkyle, halogénalkoxy ou halogénalkylthio à chaîne droite ou ramifiée ayant chacun 1 à 9 atomes de carbone et, le cas échéant, 1 à 9 atomes d'halogènes identiques ou différents ; un groupe trialkylsilyle contenant 1 à 4 atomes de carbone dans les parties alkyle individuelles à chaîne droite ou à chaîne ramifiée ; un groupe cycloalkylalkyle ou cycloalkyle ayant chacun 3 à 7 atomes de carbone dans la partie cycloalkyle et, le cas échéant 1 à 4 atomes de carbone dans la partie alkyle à chaîne droite ou ramifiée et portant chacun le cas échéant un ou plusieurs substituants, identiques ou différents, alkyle ayant 1 à 4 atomes de carbone et halogéno ; un groupe alkylène ou un groupe alcénylène ayant chacun deux jonctions et jusqu'à 5 atomes de carbone et portant chacun le cas échéant un ou plusieurs substituants, identiques ou différents, alkyle, halogénalkyle, alkoxy ou halogénalkoxy avec chacun 1 à 4 atomes de carbone et, le cas échéant, 1 à 9 atomes d'halogènes identiques ou différents, et/ou halogéno, un groupe aryle à une seule jonction ou un groupe arylène à deux jonctions ayant chacun 6 à 10 atomes de carbone et portant chacun le cas échéant un ou plusieurs substituants identiques ou différents, en considérant comme substituants : un halogène, un groupe cyano, nitro, un groupe alkyle, alkoxy, alkylthio, halogénalkyle, halogénalkoxy, halogénalkylthio, alkoxycarbonyle ou alkoxyiminoalkyle ayant chacun une chaîne droite ou ramifiée avec, dans chaque cas, 1 à 4 atomes de carbone dans les parties alkyle individuelles et, le cas échéant, 1 à 9 atomes d'halogènes identiques ou différents,

Y est un halogène ou un groupe alkylsulfonyloxy ou arylsulfonyloxy éventuellement substitué, à l'exception des composés dans lesquels A[1] est une chaîne 1,4-butanediyle en même temps que Y représente le brome ou l'iode.

10. Procédé de production de tétrahydrofurannes 35 substitués de formule (IIa)

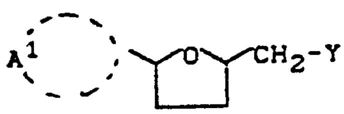

(IIa)

dans laquelle

A[1] désigne une chaîne alkylène ou une chaîne alcénylène à deux jonctions, ayant chacune 3 à 18 atomes de carbone et portant chacune le cas échéant un ou plusieurs substituants identiques ou différents, en considérant comme substituants :

un groupe alkyle, alkoxy, alkylthio, alkoxycarbonyle, halogénalkyle, halogénalkoxy ou halogénalkylthio à chaîne droite ou ramifiée ayant chacun 1 à 9 atomes de carbone et, le cas échéant, 1 à 9 atomes d'halogènes identiques ou différents ; un groupe trialkylsilyle contenant 1 à 4 atomes de carbone dans les parties alkyle individuelles à chaîne droite ou à chaîne ramifiée ; un groupe cycloalkylalkyle ou cycloalkyle ayant chacun 3 à 7 atomes de carbone dans la partie cycloalkyle et, le cas échéant 1 à 4 atomes de carbone dans la partie alkyle à chaîne droite ou ramifiée et portant chacun le cas échéant un ou plusieurs substituants, identiques ou différents, alkyle ayant 1 à 4 atomes de carbone et halogéno ; un groupe alkylène ou un groupe alcénylène ayant chacun deux jonctions et jusqu'à 5 atomes de carbone et portant chacun le cas échéant un ou plusieurs substituants, identiques ou différents, alkyle, halogénalkyle, alkoxy ou halogénalkoxy avec chacun 1 à 4 atomes de carbone et, le cas échéant, 1 à 9 atomes d'halogènes identiques ou différents, et/ou halogéno, un groupe aryle à une seule jonction ou un groupe arylène à deux jonctions ayant chacun 6 à 10 atomes de carbone et portant chacun le cas échéant un ou plusieurs substituants identiques ou différents, en considérant comme substituants : un halogène, un groupe cyano, nitro, un groupe alkyle, alkoxy, alkylthio, halogénalkyle, halogénalkoxy, halogénalkylthio, alkoxycarbonyle ou alkoxyiminoalkyle ayant chacun une chaîne droite ou ramifiée avec, dans chaque cas, 1 à 4 atomes de carbone dans les parties alkyle individuelles et, le cas échéant, 1 à 9 atomes d'halogènes identiques ou différents,

Y est un halogène ou un groupe alkylsulfonyloxy ou arylsulfonyloxy éventuellement substitué, à l'exception des composés dans lesquels A[1] est une chaîne 1,4-butanediyle en même temps que Y représente le brome ou l'iode,

caractérisé en ce qu'on fait réagir les cétones cycliques de formule (VI)

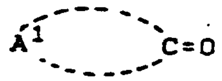

(VI)

dans laquelle

A¹ a la définition indiquée ci-dessus, tout d'abord dans une première étape avec le bromure de butène-1-yl-4-magnésium de formule (VII)

$$CH_2 = CH - CH_2 - CH_2 - Mg - Br \quad (VII)$$

en présence d'un diluant à des températures comprises entre −20°C et +50°C et on fait réagir les carbinols ainsi obtenus de formule (VIII)

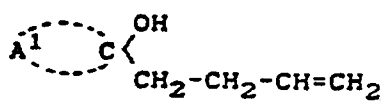

(VIII)

dans laquelle

A¹ a la définition indiquée ci-dessus, avec un agent d'halogénation, en présence de quinoléine ou avec le N-bromosuccinimide, éventuellement en présence d'un diluant, à des températures comprises entre −20°C et +80°C, ou bien on cyclise des composés trihydroxyliques de formule (IX)

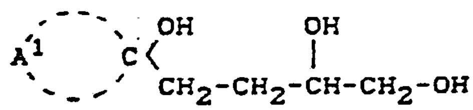

(IX)

dans laquelle

A¹ a la définition indiquée ci-dessus, d'une manière classique avec des acides et on fait réagir les hydroxyméthyltétrahydro furannes ainsi obtenus de formule (X)

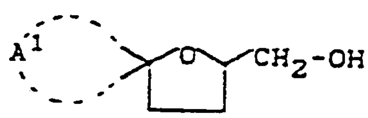

(X)

dans laquelle

A¹ a la définition indiquée ci-dessus, dans une seconde étape avec des halogénures d'acides sulfoniques de formule (XI)

$$R^3 - SO_2 - Hal \quad (XI)$$

dans laquelle

$R^3$ est un groupe alkyle éventuellement substitué par un halogène ou un groupe aryle éventuellement substitué par un groupe alkyle ayant 1 à 4 atomes de carbone et

Hal est un halogène,

le cas échéant en présence d'un diluant et en la présence éventuelle d'un accepteur d'acide, à des températures comprises entre 0 et 120°C.